# EUROPÄISCHE PATENTANMELDUNG

(11) **EP 1 905 420 A1**
(43) Veröffentlichungstag der Anmeldung: **02.04.2008**
(21) Anmeldenummer: 07117178.9
(22) Anmeldetag: 25.09.2007
(51) Int. Cl.: A61K 8/33, A61Q 13/00, A61Q 5/02, C11B 9/00, C11D 7/60

(54) **Mischungen umfassend alpha-Ambrinolalkylether und 2-Alkoxy-9-methylen-2,6,6-trimethylbicyclo[3.3.1]nonane als Riech- und Aromastoffe**

(30) Priorität: 28.09.2006 DE 102006046249
(71) Anmelder: Symrise GmbH & Co. KG, 37603 Holzminden (DE)
(72) Erfinder: Hölscher, Bernd, 37620, Halle (DE)
(74) Vertreter: Eisenführ, Speiser & Partner

(57) **Zusammenfassung**

Beschrieben wird eine Mischung umfassend oder bestehend aus
(a) einer oder mehreren Verbindungen der Formel (I) 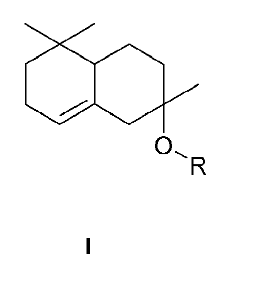 und
(b) einer oder mehreren Verbindungen der Formel (II) 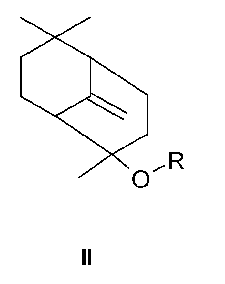 wobei
R in Formel (I) und Formel (II) die gleiche oder eine unterschiedliche Bedeutung besitzt und jeweils ausgewählt ist aus der Gruppe bestehend aus Methyl und Ethyl, sowie gegebenenfalls
(c) einem, zwei oder mehr weiteren Riech- oder Aromastoffen.

## Beschreibung

Die vorliegende Erfindung betrifft primär Mischungen bestimmter alpha-Ambrinolalkylether und 2-Alkoxy-9-methylen-2,6,6-trimethylbicyclo[3.3.1]nonane, die als Riech- und Aromastoffmischungen verwendet werden können. Die Erfindung betrifft ferner die entsprechende Verwendung einer erfindungsgemäßen Mischung als Riech- oder Aromastoffmischung, die Verwendung bestimmter 2-Alkoxy-9-methylen-2,6,6-trimethylbicyclo[3.3.1]nonane als Mittel zum Maskieren einer unangenehmen Geruchsnote, die Verwendung der besagten alpha-Ambrinolalkylether zum Modifizieren der geruchlichen Eigenschaften der besagten 2-Alkoxy-9-methylen-2,6,6-trimethylbicyclo[3.3.1]nonane, entsprechende parfümierte Produkte, entsprechende Verfahren zum Vermitteln, Modifizieren und/oder Verstärken eines Geruches sowie Verfahren zur Herstellung der besagten alpha-Ambrinolalkylether und der besagten Mischungen. Weitere Aspekte der vorliegenden Erfindung ergeben sich aus der nachfolgenden Beschreibung und den beigefügten Patentansprüchen.

Trotz einer Vielzahl bereits vorhandener Riechstoffe besteht in der Parfümindustrie auch weiterhin ein genereller Bedarf an neuen Riechstoffen. So besteht ein Bedarf an Riechstoffen mit holzigen, ambrierenden Duftnoten, die in der Lage sind, (in Riechstoffkompositionen) neben einer holzigen, ambrierenden Duftnote weitere interessante Geruchsnoten zu erzeugen und mit ihren neuartigen bzw. originellen Dufteigenschaften die Möglichkeiten des Parfümeurs zu erweitern. Insbesondere besteht ein Interesse an Riechstoffen mit holzigen, ambrierenden Duftnoten, welche in der Lage sind, eine harmonische Kombination mit blumig duftenden Riechstoffen einzugehen. Vorzugsweise sollte eine Überlagerung der unterschiedlichen geruchlichen Aspekte und Noten erfolgen, um dadurch einen insgesamt komplexen Geruchseindruck zu erzeugen.

Für die Kreation neuartiger moderner Kompositionen besteht ständiger Bedarf an Riechstoffen mit besonderen geruchlichen Eigenschaften, die geeignet sind, als Grundlage für die Komposition von neuartigen modernen Parfüms mit komplexen Geruchscharakter zu dienen. Bevorzugte gesuchte Riechstoffe sollten daher neben einer holzigen, ambrierenden Duftnote weitere Noten und Aspekte aufweisen, die ihnen geruchlichen Charakter und Komplexität verleihen.

Die Suche nach geeigneten Riech- und Aromastoffen, die zur vorliegenden Erfindung führte, wurde durch folgende Sachverhalte erschwert:
- Die Mechanismen der Geruchswahrnehmung sind nicht ausreichend bekannt.
- Die Zusammenhänge zwischen der speziellen Geruchswahrnehmung einerseits und der chemischen Struktur des zugehörigen Riechstoffs andererseits sind nicht hinreichend erforscht.
- Häufig bewirken bereits geringfügige Änderungen am strukturellen Aufbau eines bekannten Riechstoffs starke Änderungen der sensorischen Eigenschaften und beeinträchtigen die Verträglichkeit für den menschlichen Organismus.

Der Erfolg der Suche nach geeigneten Riechstoffen hängt deshalb stark von der Intuition des Suchenden ab.

Daher bestand die dieser Erfindung zugrunde liegende Aufgabe im Wesentlichen darin, Riech- bzw. Aromastoffe mit holzigen, ambrierenden Duftnoten zu finden, welche gepaart sind mit weiteren interessanten und originellen geruchlichen Eigenschaften, wodurch die gesuchten Riechstoffe neuartige und originelle Riechstoffkompositionen mit besonderen geruchlichen Noten und Aspekten ermöglichen. Insbesondere sollten Riechstoffe mit holzigen, ambrierenden Duftnoten gefunden werden, welche insbesondere zur Kombination mit Riechstoffen geeignet sind, welche eine blumige Duftnote aufweisen.

Daneben sollten die diese Hauptaufgabe erfüllenden Riechstoffe ferner vorzugsweise über ihre primären, nämlich geruchlichen, Eigenschaften hinaus zusätzliche positive Sekundäreigenschaften besitzen, wie z. B. eine höhere Stabilität unter bestimmten Anwendungsbedingungen, eine höhere Ausgiebigkeit, ein besseres Haftungsvermögen, eine hohe Substantivität, eine bemerkenswerte Booster-Wirkung oder ein starkes Blooming, so dass sensorisch bemerkenswerte Effekte oder aber auch bessere dermatologische und toxikologische Eigenschaften gegenüber vergleichbaren Riechstoffen erzielt werden können.

Erfindungsgemäß gelöst wird die primäre gestellte Aufgabe durch Angabe einer Mischung umfassend oder bestehend aus
(a) einer oder mehreren Verbindungen der Formel (I) 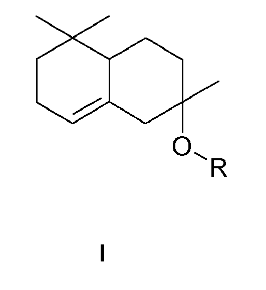 und
(b) einer oder mehreren Verbindungen der Formel (II) 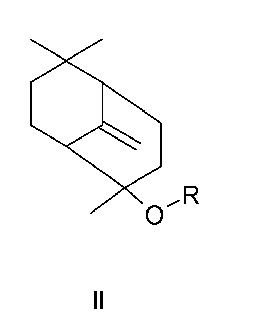 wobei
   R in Formel (I) und Formel (II) die gleiche oder eine unterschiedliche Bedeutung besitzt und jeweils ausgewählt ist aus der Gruppe bestehend aus Methyl und Ethyl,
   sowie gegebenenfalls
(c) einem, zwei oder mehr weiteren Riech- oder Aromastoffen.

Die als Komponente (a) eingesetzten Verbindungen der Formel (I) sind alpha-Ambrinolmethyl- oder -ethylether, die sich formal von alpha-Ambrinol ableiten.

Die Verbindungen der Formel (I) besitzen ebenso wie alpha-Ambrinol zwei asymmetrische Kohlenstoffatome. Die Verbindungen der Formel (I) können als (R,R)-konfiguriertes Enantiomer, (R,S)-konfiguriertes Enantiomer, (S,R)-konfiguriertes Enantiomer, (S,S)-konfiguriertes Enantiomer oder als beliebiges Gemisch von Enantiomeren, insbesondere als Racemat, oder auch als beliebiges Gemisch der entsprechenden Diastereomeren, vorliegen. Sofern zur Herstellung einer Verbindung der Formel (I) (zu Herstellverfahren siehe auch unten) von enantiomerenreinem oder enantiomerenangereichertem alpha-Ambrinol ausgegangen wird, erhalten sich die jeweiligen Konfigurationen und es entsteht enantiomerenreiner bzw. enantiomerenangereicherter alpha-Ambrinolalkylether. Hinsichtlich der den alpha-Ambrinolalkylethern zugrunde liegenden alpha-Ambrinole sei verwiesen auf: Ohloff, G., Fragrance Chemistry, Seite 546, Academic Press, New York; 1982; P. Naegeli and Y. Wirz-Habersack, Enantiodifferentiation of odour perception of alphaambrinols, Tetrahedron: Asymmetry, Volume 3, Issue 2, 1992, Seiten 221-222.

Die als Komponente (b) eingesetzten Verbindungen der Formel (II) sind 2-Alkoxy-9-methylen-2,6,6-trimethylbicyclo[3.3.1]nonane (wobei hier Alkoxy Methoxy oder Ethoxy bedeutet).

Sofern als Komponente (c) ein, zwei oder mehr weitere Riechstoffe vorhanden sind, besitzen einer, zwei oder mehr dieser Duftstoffe vorzugsweise eine holzige und/oder blumige Duftnote.

Die erfindungsgemäßen Mischungen besitzen eine überraschend komplexe holzige, ambrierende Note, vergleiche hierzu die detaillierteren Geruchsbeschreibungen weiter unten. Die erfindungsgemäßen Mischungen sind deshalb hervorragend als Riechstoff- oder Aromastoffmischungen verwendbar.

Über ihre primären, nämlich geruchlichen, Eigenschaften hinaus verfügen die erfindungsgemäßen Mischungen zusätzlich über positive Sekundäreigenschaften. Insbesondere in sauren Anwendungen besitzen die erfindungsgemäßen Mischungen einen erheblichen Stabilitätsvorteil gegenüber Riechstoffen mit ähnlichen Geruchseigenschaften.

In Helv. Chim. Acta 1955, 38, 1573 wird über die Cyclisierung von alpha- Dihydrojonon zu einer Verbindung der obigen Formel (II) mit R = Ethyl berichtet. Der Geruch dieser Verbindung wird - entsprechend dem der unveretherten Alkohole - als warm holzig-ambriert, zum Teil mit einer mehr oder weniger stark ausgeprägten camphrigen Note beschrieben.

In der europäischen Patentschrift EP 0 330 995 B1 werden Verbindungen der obigen Formel (II) beschrieben, die als Gemische entsprechender exo- und endo-Formen vorliegen. Zu diesen Gemischen der exo- und endo-Formen wird ausgeführt, dass sie sich durch kräftige und sehr natürlich-warme Noten in Richtung Tabak, Ambergris und Holz auszeichnen. Das exo-Isomere wird als sensorisch interessanter beschrieben.

Der Ethylether (obige Verbindung der Formel (II) mit R = Ethyl) steht gemäß der EP 0 330 995 B1 im Vordergrund des Interesses und besitzt einen holzigen, ambraartigen, blumigen Geruch (Richtung Iriswurzel) mit Aspekten von Patchouli, Muskatellersalbei und Tabak. Der Methylether soll ähnliche Geruchseigenschaften aufweisen.

Die US-Patentschrift US 2,803,662 beschreibt ebenfalls Verbindungen der obigen Formel (II).

Die Verbindung der Formel (II) mit R = Ethyl, d.h. die Verbindung 2-Ethoxy-9-methylen-2,6,6-trimethylbicyclo[3.3.1]nonan besitzt die CAS-Nummer 68845-00-1 und ist im Handel erhältlich.

In der Veröffentlichung WO 82/00030 (Willis et al) wird unter anderem die Herstellung von alpha-Ambrinolmethylether (d.h. einer Verbindung der obigen Formel (I) mit R = Methyl) über die Veretherung von alpha-Ambrinol mit Methyljodid beschrieben. Eine sensorische Beschreibung des Ethers wurde jedoch nicht angegeben.

In eigenen Untersuchungen zeigte sich, dass die Verbindungen der Formel (I), d.h. alpha-Ambrinolmethylether und alpha-Ambrinolethylether einen unangenehmen Geruch besitzen; dieser Geruch ließ zunächst einen Einsatz in Riechstoffmischungen als ausgeschlossen erscheinen. Die Geruchsbeschreibungen lauten wie folgt:
1. alpha-Ambrinolmethylether (Verbindung der Formel (I) mit R = Methyl): stark animalisch, fäkalisch, Indol/Skatol, ähnlich alpha-Ambrinol
2. alpha-Ambrinolethylether (Verbindung der Formel (I) mit R = Ethyl): animalisch, phenolisch, erinnert an Styrax.

Überraschenderweise sind die erfindungsgemäßen Mischungen aber trotz der animalischen bzw. animalisch-fäkalischen Geruchsnoten der alpha-Ambrinolalkylether hervorragend zum Einsatz als Riechstoff geeignet. Es zeigten sich nämlich in eigenen Untersuchungen zwei unerwartete sensorische Effekte, die auf einer derzeit nicht erklärbaren Wechselwirkung zwischen den Verbindungen der Formel (I) und den Verbindungen der Formel (II) zu basieren scheinen:

Zum einen sind überraschender Weise die Verbindungen der Formel (II) in der Lage, unangenehme Geruchsnoten, welche ansonsten z. B. von den Verbindungen der Formel (I) verursacht werden, zu maskieren. Insbesondere die stark animalischen und fäkalischen Geruchsnoten des alpha-Ambrinolmethylether bzw. die animalische Geruchsnote des alpha-Ambrinolethylether werden durch die Verbindungen der Formel (II) maskiert. Die resultierende erfindungsgemäße Mischung besitzt somit keine von den Ambrinolethern ausgehenden animalischen bzw. fäkalischen Geruchsnoten bzw. bei Einsatz einer nur geringen Menge an Verbindungen der Formel (II) im Vergleich zur vorhandenen Menge an Verbindungen der Formel (I) lediglich eine animalische bzw. fäkalische Rest-Geruchsnote, deren Stärke geringer ist als aufgrund des Anteils von Verbindungen der Formel (I) in der Mischung eigentlich zu erwarten gewesen wäre.

Zum anderen sind die Verbindungen der Formel (I) in der Lage, die geruchlichen Eigenschaften von Verbindungen der Formel (II) (mit gleicher oder unterschiedlicher Bedeutung von R) in Richtung strahlender, ambrierender zu modifizieren.

Insgesamt ergibt sich der Eindruck, dass in Mischungen der Verbindungen der Formel (I) und der Verbindungen der Formel (II) die unangenehmen animalisch-fäkalischen Geruchseindrücke verschwinden, welche ansonsten von den alpha- Ambrinolalk y-lethern der Formel (I) vermittelt werden und stattdessen der Geruchseindruck der Verbindungen der Formel (II) strahlender und ambrierender wird. Die vorliegende Erfindung betrifft auch entsprechende Verwendungen.

In den erfindungsgemäßen Mischungen wirken die Verbindungen der Formel (I) also nicht so, wie man aufgrund der zugehörigen Geruchsbeschreibungen der Reinstoffe erwarten könnte. Und auch die Verbindungen der Formel (II) leisten mehr, als der Fachmann angesichts des Standes der Technik erwartet hätte, denn sie fungieren nicht nur als Riechstoff, sondern zugleich auch als Maskierungsmittel.

Über ihre primären, nämlich geruchlichen, Eigenschaften hinaus verfügen die erfindungsgemäßen Mischungen umfassend Verbindungen der Formeln (I) und (II) über zusätzliche positive Sekundäreigenschaften. Insbesondere in sauren Anwendungen haben die erfindungsgemäßen Mischungen eine erheblich höhere Stabilität als z. B. alpha-Ambrinol.

Bevorzugte erfindungsgemäße Mischungen umfassen oder bestehen aus
- jeweils einer Verbindung der Formel (I) und einer Verbindung der Formel (II) mit gleicher Bedeutung von R
   sowie entweder
   - einer weiteren Verbindung der Formel (I) mit der anderen Bedeutung von R und/oder
      einer weiteren Verbindung der Formel (II) mit der anderen Bedeutung von R
      oder
   - keiner weiteren Verbindung der Formel (I) oder (II).

Bevorzugte erfindungsgemäße Mischungen umfassen oder bestehen also beispielsweise aus einer Verbindung der Formel (I) in der R Methyl bedeutet, und einer Verbindung der Formel (II), in der R Methyl bedeutet, oder sie umfassen oder bestehen aus einer Verbindung der Formel (I), in der R Ethyl bedeutet, und einer Verbindung der Formel (II), in der R Ethyl bedeutet. Vorzugsweise bedeutet R jedoch sowohl in Formel (I) als auch in Formel (II) Methyl.

Insbesondere die erfindungsgemäßen Mischungen umfassend alpha-Ambrinolmethylether (Verbindung der Formel (I) mit R = Methyl) und 2-Methoxy-9-methylen-2,6,6-trimethylbicyclo[3.3.1]nonan (Verbindung der Formel (II) mit R = Methyl) vermitteln einen sehr komplexen und vielfältigen Geruchs- und Geschmackseindruck, der sonst nur durch komplexe Mischungen mehrerer Komponenten erreicht werden kann (wie beispielsweise durch ätherische Öle oder Kräuter- bzw. Gewürzmischungen).

Bevorzugt ist der Einsatz von reinen exo-Isomeren der Formel (II) oder von exo/endo-Mischungen mit einem größeren Anteil der exo-Form.

Sofern nicht im Einzelfall eine gewisse animalische oder gar fäkalische Geruchsnote gewünscht ist, wird in einer erfindungsgemäßen Mischung die Gesamtmenge an Verbindungen der Formel (II) so ausgewählt, dass eine ansonsten von den Verbindungen der Formel (I) verursachte animalische Geruchsnote wirksam maskiert ist.

Vorzugsweise liegt das Verhältnis der Gesamtmenge von Verbindungen der Formel (I) in einer erfindungsgemäßen Mischung zur Gesamtmenge von Verbindungen der Formel (II) im Bereich von 100 : 1 bis 1 : 10 liegt, bevorzugt im Bereich von 3 : 1 bis 1 : 3, besonders bevorzugt im Bereich von 2 : 1 bis 1 : 1.

Besonders bevorzugte erfindungsgemäße Mischungen ergeben sich aus der nachfolgenden Tabelle.

| Angabe in Gew.-% Ether der Formel I | Angabe in Gew.-% Ether der Formel II exo/endo ca. 68:32 | jeweils Anzahl C-Atome in R |
|---|---|---|
| 50% | 50% | 1 |
| 50% | 50% | 2 |
| 55% | 45% | 1 |
| 55% | 45% | 2 |
| 75% | 25% | 1 |
| 75% | 25% | 2 |
| 25% | 75% | 1 |
| 25% | 75% | 2 |

Die Geruchseigenschaften der in Tabelle 1 angegebenen bevorzugten erfindungsgemäßen Mischungen werden jeweils wie folgt beschrieben: sehr stark strahlend, ambrierend, exaltierend, weich holzig, erinnert an graue Ambra und Civet, sehr natürlicher Geruchseindruck. Die erfindungsgemäßen Mischungen sind somit neue und wertvolle Riechstoffmischungen, die sich in ihren eigenständigen olfaktorischen Eigenschaften deutlich von denen bekannter Riechstoffe anheben und diese übertreffen.

Bei Einsatz von weniger als 25 Gew.-% 2-Methoxy-9-methylen-2,6,6-trimethylbicyclo[3.3.1]nonan (Verbindung der Formel (II) mit R = Methyl) in einer Mischung, die außerdem lediglich alpha-Ambrinolmethylether (Verbindung der Formel (I) mit R = Methyl) umfasst, entfallen die unangenehmen animalisch-fäkalischen Geruchseigenschaften der Verbindung der Formel (I) nicht mehr vollständig, was allerdings im Einzelfall gewünscht sein kann. Bei Anwesenheit von mindestens 25 Gew.-% 2-Methoxy-9-methylen-2,6,6-trimethylbicyclo[3.3.1]nonan entfallen die unangenehmen animalisch-fäkalischen Geruchseigenschaften und die Mischung riecht für den Parfümeur heller, sehr stark strahlend, ambrierender, exaltierend, weich holzig, und vermittelt einen sehr natürlichen Geruchseindruck der an graue Ambra und Civet erinnert. Bei einem Anteil von über 75 Gew.-% 2-Methoxy-9-methylen-2,6,6-trimethylbicyclo[3.3.1]nonan riecht eine erfindungsgemäße Mischung dann wieder schwächer und erreicht mit zunehmendem Anteil der Verbindung der Formel (II) nicht mehr einen sehr stark strahlenden, ambrierenden, exaltierenden Geruchseindruck.

Es versteht sich, dass bevorzugte erfindungsgemäße Mischungen einzelne, mehrere oder sämtliche der folgenden geruchlichen Eigenschaften aufweisen: sehr stark strahlend, ambrierend, exaltierend, weich holzig, erinnert an graue Ambra und Civet, sehr natürlicher Geruchseindruck.

Die vorliegende Erfindung betrifft auch die Verwendung einer erfindungsgemäßen Mischung wie vorstehend beschrieben (insbesondere in einer vorstehend als bevorzugt bezeichneten Ausgestaltung) als Riechstoff- oder Aromastoffmischung, insbesondere als Riechstoff mit einer, mehreren oder sämtlichen der folgenden geruchlichen Eigenschaften: sehr stark strahlend, ambrierend, exaltierend, weich holzig, erinnert an graue Ambra und Civet, sehr natürlicher Geruchseindruck.

Gemäß einem verwandten Aspekt betrifft die vorliegende Erfindung auch ein Verfahren zum Vermitteln, Modifizieren und/oder Verstärken eines Geruches, mit folgendem Schritt: Kontaktieren oder Vermischen eines Erzeugnisses mit einer sensorisch wirksamen Menge einer erfindungsgemäßen Mischung.

Vorzugsweise wird im Rahmen der erfindungsgemäßen Verwendung bzw. des erfindungsgemäßen Verfahrens eine erfindungsgemäße Mischung eingesetzt, bei der die Gesamtmenge an Verbindungen der Formel (II) so ausgewählt ist, dass eine ansonsten von den Verbindungen (I) verursachte animalische Geruchsnote wirksam maskiert ist. Hinsichtlich der insoweit vorzugsweise einzustellenden Mengenverhältnisse sei auf die obigen Ausführungen verwiesen.

Die erfindungsgemäßen Mischungen umfassen üblicherweise eine sensorisch wirksame Gesamtmenge an den Verbindungen der Formeln (I) und (II). Neben den Verbindungen der Formeln (I) und (II) sind häufig ein, zwei oder mehr weitere Riech- oder Aromastoffe in einer erfindungsgemäßen Mischung vorhanden. Das Gewichtsverhältnis der Gesamtmenge an erfindungsgemäß einzusetzenden Verbindungen der Formeln (I) und (II) zu der Gesamtmenge an weiteren Riech- oder Aromastoffen liegt vorzugsweise im Bereich von 1 : 1000 bis 1:0,50.

Besonders bevorzugt umfasst eine erfindungsgemäße Mischung als Komponente (c) einen, zwei oder mehr weitere holzige Riechstoffe (incl. Sandel, Moschus und Ambra), die ausgewählt sind aus der Gruppe bestehend aus: Sandranol (2-Ethyl-4-(2,2,3)-trimethylcyclopent-3-yl-but-2-en-l-ol), Ysamber K (1',1',5',5'-Tetramethylhexahydrospiro[1,3-dioxoian-2,8'(5'H)-2H-2,4a-methanonaphthalen]), Globanon (Cyclohexadec-8-en-1-on), Timberol (1-(2,2,6-Trimethylcyclohexyl)hexan-3-ol), Iso-E-Super (2,3,8,8,-Tetramethyl-1,2,3,4,5,6,8-octahydro-2-naphthalenyl-methylketon), Cashmeran (1,1,2,3,3-Pentamethyl TH-Indan-4-on/6,7-dihydro-1,1,2,3,3-pentamethyl-4(5H)-indanon), Isobornylacetat (2-exo-Bornanylacetat), Ylanat (2-tert-Butylcyclohexylacetat).

Die Kombination einer Mischung der erfindungsgemäß einzusetzenden Verbindungen der Formeln (I) und (II) mit den genannten holzigen Riechstoffen führt zu helleren, strahlenderen und sauberen Geruchseindrücken. Die entsprechenden erfindungsgemäßen Mischungen wirken natürlicher und frischer.

Ebenfalls besonders bevorzugt ist eine erfindungsgemäße Mischung umfassend einen, zwei oder mehr blumige Riechstoffe ausgewählt aus der Gruppe best ehend aus: Lilial (2-Methyl-3-(4-tert-butylphenyl)propanal), Hedion (Methyl (3-oxo-2-pentylcyclopentyl)acetat), Mayol (4-Isopropyl-cyclohexyl) methanol), Linalool (3,7 - Dimethyl-1,6-octadien-3-ol), Dihydromyrcenol (2,6-Dimethyl-7-octen-2-ol), Citronellol (3,7 -Dimethyl-6-octen-1-ol), Phenoxanol (3-Methyl-5-Phenyl-pentanol), 2-Phenylethylalkohol, Hydroxycitronellal (3,7 -Dimethyl-7-hydroxyoctan-1-al), alpha-Jonon (4-(2,6,6-Trimethyl-cyclohex-2-enyl)-but-3-en-2-on).

Die Kombination erfindungsgemäß einzusetzender Verbindungen der Formeln (I) und (II) mit oben genannten blumigen Riechstoffen führt zu einer Modifikation in Richtung Frische und Ausstrahlung. Zudem werden die blumigen Aspekte abgerundeter. Die Mischungen riechen intensiver und harmonischer.

Des Weiteren bevorzugt ist die Anwesenheit zumindest eines holzigen Riechstoffs aus der vorstehend genannten Gruppe und zumindest eines blumigen Riechstoffs aus der vorstehend genannten Gruppe.

Neben den genannten Kombinationen mit holzigen und/oder blumigen Riechstoffen ist vielfach eine Kombination der erfindungsgemäß einzusetzenden Verbindungen der Formeln (I) und (II) mit grün-fruchtigen Riechstoffen besonders bevorzugt.

Geeignete grün-fruchtige Riechstoffe sind beispielsweise: Vertral (Octahydro-1H-4,7-methanoinden-5-carbaldehyd), cis-3-Hexen-1-ol, beta-Damascon (1-(2,6,6-Trimethylcyclohex-2-enyl)-buten-1-on), Vertocitral (2,4-Dimethylcyclohex-3-en-1-carbaldehyd), Cyclogalbanat (Allyl (Cyclohexyloxy) acetat), Hexylacetat.

Die Kombination mit den genannten grün-fruchtigen Riechstoffen führt zu einem runderen und weicheren Geruch. Zudem verleiht der Zusatz der erfindungsgemäß einzusetzenden Verbindungen der Formeln (I) und (II) zu den grün-fruchtigen Riechstoffen einen gewissen Glanz und vermittelt einen Eindruck von natürlicher Ausstrahlung.

Häufig ist auch eine Kombination der erfindungsgemäß einzusetzenden Verbindungen der Formeln (I) und (II) mit würzig-balsamischen Riechstoffen vorteilhaft. Geeignete würzig-balsamische Riechstoffe sind insbesondere: Eugenol (2-Methoxy-4-allylphenol), Cumarin (2H-1-Benzopyran-2-on), Anisaldehyd (4-Methoxybenzaldehyd), Amylzimtaldehyd (2-Pentyl-3-phenyl-2-propenal), Isoamylsalicylat (Salicylsäure-3-methylbutylester) und Zimtalkohol (3-Phenyl-2-propen-1-ol).

Bei der Kombination der erfindungsgemäßen zu verwendenden Verbindungen der Formeln (I) und (II) mit den genannten würzig-balsamischen Riechstoffen sind Aspekte von Frische und Natürlichkeit zu beobachten. Die Mischungen wirken harmonischer und strahlender.

Ganz allgemein lassen sich die erfindungsgemäß einzusetzenden Verbindungen der Formel (I) und Verbindungen der Formel (II) mit einer Vielzahl von Riechstoffen vorteilhaft zu neuen Riech- oder Aromastoffkompositionen kombinieren. Riechstoffe (als Komponente (c) einer erfindungsgemäßen Mischung), die zur Kombination vorteilhafterweise geeignet sind, finden sich z. B. in S. Arctander, Perfume and Flavor Materials, Vol. I und II, Montclair, N. J. 1969, Eigenverlag, oder K. Bauer et al., Common Fragrance and Flavor Materials, 4th Edition, Wiley-VCH, Weinheim 2001. Im einzelnen seien genannt:

Extrakte aus natürlichen Rohstoffen wie Etherische Öle, Concretes, Absolues, Resine, Resinoide, Balsame, Tinkturen wie z. B.

Ambratinktur; Amyrisöl; Angelicasamenöl; Angelicawurzelöl; Anisöl; Baldrianöl; Basilikumöl; Baummoos -Absolue; Bayöl; Beifußöl; Benzoeresin; Bergamotteöl; Bienenwachs-Absolue; Birkenteeröl; Bittermandelöl; Bohnenkrautöl; Buccoblätteröl; Cabreuvaöl; Cadeöl; Calmusöl; Campheröl; Canangaöl; Cardamomenöl; Cascarillaöl; Cassiaöl; Cassie-Absolue; Castoreum-absolue; Cedernblätteröl; Cedernholzöl; Cistusöl; Citronellöl; Citronenöl; Copaivabalsam; Copaivabalsamöl; Corianderöl; Costuswurzelöl; Cuminöl; Cypressenöl; Davanaöl; Dillkrautöl; Dillsamenöl; Eau de brouts-Absolue; Eichenmoos-Absolue; Elemiöl; Estragonöl; Eucalyptus-citriodora-Öl; Eucalyptusöl; Fenchelöl ; Fichtennadelöl; Galbanumöl; Galbanumresin; Geraniumöl; Grapefruitöl; Guajakholzöl; Gurjunbalsam; Gurjunbalsamöl; Helichrysum-Absolue; Helichrysumöl; Ingweröl; Iriswurzel-Absolue; Iriswurzelöl; Jasmin-Absolue; Kalmusöl; Kamillenöl blau; Kamillenöl römisch; Karottensamenöl; Kaskarillaöl; Kiefernadelöl; Krauseminzöl; Kümmelöl; Labdanumöl; Labdanum-Absolue; Labdanumresin; Lavandin-Absolue; Lavandinöl ; Lavendel-Absolue; Lavendelöl; Lemongrasöl; Liebstocköl; Limetteöl destilliert; Limetteöl gepreßt; Linaloeöl; Litsea-cubeba-Öl; Lorbeerblätteröl; Macisöl; Majoranöl; Mandarinenöl; Massoirindenöl; Mimosa-Absolue; Moschuskörneröl; Moschustinktur; Muskateller-Salbei-ÖI; Muskatnußöl; Myrrhen-Absolue; Myrrhenöl; Myrtenöl; Nelkenblätteröl; Nelkenblütenöl; Neroliöl; Olibanum-Absolue; Olibanumöl; Opopanaxöl; Orangenblüten-Absolue; Orangenöl; Origanumöl; Palmarosaöl; Patchouliöl; Perillaöl; Perubalsamöl; Petersilienblätteröl; Petersiliensamenöl; Petitgrainöl; Pfefferminzöl; Pfefferöl; Pimentöl; Pineöl (pine oil); Poleyöl; Rosen-Absolue; Rosenholzöl; Rosenöl; Rosmarinöl; Salbeiöl dalmatinisch; Salbeiöl spanisch; Sandelholzöl; Selleriesamenöl; Spiklavendelöl; Sternanisöl; Styraxöl; Tagetesöl; Tannennadelöl; Teatree-Öl; Terpentinöl; Thymianöl; Tolubalsam; Tonka-Absolue; Tuberosen-Absolue; Vanilleextrakt; Veilchenblätter-Absolue; Verbenaöl; Vetiveröl; Wacholderbeeröl; Weinhefenöl; Wermutöl; Wintergrünöl; Ylangöl; Ysopöl; Zibet-Absolue; Zimtblätteröl; Zimtrindenöl sowie Fraktionen davon, bzw. daraus isolierten Inhaltsstoffen;

Einzel-Riechstoffe aus der Gruppe der Kohlenwasserstoffe, wie z. B. 3-Caren; α-Pinen; β-Pinen; α-Terpinen; γ-Terpinen; p-Cymol; Bisabolen; Camphen; Caryophyllen; Cedren; Farnesen; Limonen; Longifolen; Myrcen; Ocimen; Valencen; (E,Z)-1,3,5-Undecatrien; Styrol; Diphenylmethan;

der aliphatischen Alkohole wie z. B. Hexanol; Octanol; 3-Octanol; 2,6-Dimethylheptanol; 2-Methyl-2-heptanol; 2-Methyl-2-octanol; (E)-2-Hexenol; 1-Octen-3-ol; Gemisch von 3,4,5,6,6-Pentamethyl-3/4-hepten-2-ol und 3,5,6,6-Tetramethyl-4-methyleneheptan-2-ol; (E,Z)-2,6-Nonadienol; 3,7-Dimethyl-7-methoxyoctan-2-ol; 9-Decenol; 10-Undecenol; 4-Methyl-3-decen-5-ol;

der aliphatischen Aldehyde und deren Acetale wie z. B. Hexanal; Heptanal; Octanal; Nonanal; Decanal; Undecanal; Dodecanal; 2-Methyloctanal; 2-Methylnonanal; (E)-2-Hexenal; (Z)-4-Heptenal; 2,6-Dimethyl-5-heptenal; 10-Undecenal; (E)-4-Decenal; 2-Dodecenal;2,6,10-Trimethyl-9-undecenal; 2,6,10-Trimethyl-5,9-undecadienal; Heptanaldiethylacetal; 1,1-Dimethoxy-2,2,5-trimethyl-4-hexen; Citronellyloxyacetaldehyd; 1-(1-Methoxy-propoxy)-(E/Z)-3-hexen;
der aliphatischen Ketone und deren Oxime wie z. B. 2-Heptanon; 2-Octanon; 3-Octanon; 2-Nonanon; 5-Methyl-3-heptanon ; 5-Methyl-3-heptanonoxim; 2,4,4,7-Tetramethyl-6-octen-3-on; 6-Methyl-5-hepten-2-on;
der aliphatischen schwefelhaltigen Verbindungen wie z. B. 3-Methylthio-hexanol; 3-Methylthiohexylacetat; 3-Mercaptohexanol; 3-Mercaptohexylacetat; 3-Mercapto hexylbutyrat; 3-Acetylthiohexylacetat; 1-Menthen-8-thiol;
der aliphatischen Nitrile wie z. B. 2-Nonensäurenitril; 2-Undecensäurenitril; 2-Tridecensäurenitril; 3,12-Tridecadiensäurenitril; 3,7-Dimethyl-2,6-octadien-säurenitril; 3,7-Dimethyl-6-octensäurenitril;
der Ester von aliphatischen Carbonsäuren wie z. B. (E)- und (Z)-3-Hexenylformiat; Ethylacetoacetat; Isoamylacetat; 3,5,5-Trimethylhexylacetat; 3-Methyl-2-butenylacetat; (E)-2-Hexenylacetat; (E)- und (Z)-3-Hexenylacetat; Octylacetat; 3-Octylacetat; 1-Octen-3-ylacetat; Ethylbutyrat; Butylbutyrat, ; Isoamylbutyrat; Hexylbutyrat; (E)- und (Z)-3-Hexenyl-isobutyrat; Hexylcrotonat; Ethylisovalerianat; Ethyl-2-methylpentanoat; Ethylhexanoat; Allylhexanoat; Ethylheptanoat; Allylheptanoat; Ethyloctanoat; Ethyl-(E,Z)-2,4-decadienoat; Methyl-2-octinat; Methyl-2-noninat; Allyl-2-isoamyloxyacetat; Methyl-3,7-dimethyl-2,6-octadien-oat;4-Methyl-2-pentyl-crotonat;
der acyclischen Terpenalkohole wie z. B. Geraniol; Nerol; Lavadulol; Nerolidol; Farnesol; Tetrahydrolinalool; Tetrahydrogeraniol; 2,6-Dimethyl-7-octen-2-ol; 2,6-Dimethyloctan-2-ol; 2-Methyl-6-methylen-7-octen-2-ol; 2,6-Dimethyl-5,7-octadien-2-ol; 2,6-Dimethyl-3,5-octadien-2-ol; 3,7-Dimethyl-4,6-octadien-3-ol; 3,7-Dimethyl-1,5,7-octatrien-3-ol 2,6-Dimethyl-2,5,7-octatrien-1-ol; sowie deren Formiate, Acetate, Propionate, Isobutyrate, Butyrate, Isovalerianate, Pentanoate, Hexanoate, Crotonate, Tiglinate und 3-Methyl-2-butenoate;
der acyclischen Terpenaldehyde und -ketone wie z. B. Citronellal;; 7-Methoxy-3,7-dimethyloctanal; 2,6,10-Trimethyl-9-undecenal; Geranylaceton; sowie die Dimethyl-und Diethylacetale von Geranial, Neral, der cyclischen Terpenalkohole wie z. B. Menthol; Isopulegol; alpha-Terpineol; Terpinenol-4; Menthan-8-ol; Menthan-1-ol; Menthan-7-ol; Borneol; Isoborneol; Linalooloxid; Nopol; Cedrol; Ambrinol; Vetiverol; Guajol; sowie deren Formiate, Acetate, Propionate, Isobutyrate, Butyrate, Isovalerianate, Pentanoate, Hexanoate, Crotonate, Tiglinate und 3-Methyl-2-butenoate;
der cyclischen Terpenaldehyde und -ketone wie z. B. Menthon; Isomenthon ; 8-Mercaptomenthan-3-on ; Carvon; Campher; Fenchon; alpha-Ionon; beta-Ionon; alphan-Methylionon; beta-n-Methylionon; alpha-Isomethylionon; beta-Isomethyl-ionon; alpha-Iron; beta-Damascenon; 1-(2,4,4-Trimethyl-2-cyclohexen-1-yl)-2-buten-1-on; 1,3,4,6,7,8a-Hexahydro-1,1,5,5-tetramethyl-2H-2,4a-methano-naphthalen-8(5H)-on;2-Methyl-4-(2,6,6-trimethyl-1-cyclohexen-1-yl)-2-butenal; Nootkaton ; Dihydronootkaton ; 4,6,8-Megastigmatrien-3-on; alpha-Sinensal ; beta-Sinensal ; acetyliertes Cedernholzöl (Methylcedrylketon);
der cyclischen Alkohole wie z. B. 4-tert.-Butylcyclohexanol ; 3,3,5-Trimethylcyclohexanol; 3-Isocamphylcyclohexanol; 2,6,9-Trimethyl-Z2,Z5,E9-cyclododecatrien-1-ol; 2-Isobutyl-4-methyltetrahydro-2H-pyran-4-ol;
der cycloaliphatischen Alkohole wie z. B. alpha,3,3-Trimethylcyclohexylmethanol;1-(4-Isopropylcyclohexyl)ethanol; 2-Methyl-4-(2,2,3-trimethyl-3-cyclopent-1-yl)butanol; 2-Methyl-4-(2,2,3-trimethyl-3-cyclopent-1-yl)-2-buten-1-ol; 3-Methyl-5-(2,2,3-trimethyl-3-cyclopent-1-yl)-pentan-2-ol; 3-Methyl-5-(2,2,3-trimethyl-3-cyclopent-1-yl)-4-penten-2-ol; 3,3-Dimethyl-5-(2,2,3-trimethyl-3-cyclopent-1-yl)-4-penten-2-ol; 1-(2,2,6-Trimethylcyclohexyl)pentan-3-ol; 1-(2,2,6-Trimethylcyclohexyl)hexan-3-ol;
der cyclischen und cycloaliphatischen Ether wie z. B. Cineol; Cedrylmethylether; Cyclododecylmethylether;1,1-Dimethoxycyclododecan; (Ethoxymethoxy)cyclododecan; alpha-Cedrenepoxid; 3a,6,6,9a-Tetramethyldodecahydro-naphtho[2,1-b]furan; 3a-Ethyl-6,6,9a-trimethyldodecahydronaphtho[2,1-b]furan; 1,5,9-Tri-methyl-13-oxabicyclo[10.1.0]trideca-4,8-dien; Rosenoxid; 2-(2,4-Dimethyl-3-cyclohexen-1-yl)-5-methyl-5-(1-methylpropyl)-1,3-dioxan;
der cyclischen und makrocyclischen Ketone wie z. B. 4-tert.-Butylcyclohexanon; 2,2,5-Trimethyl-5-pentylcyclopentanon; 2-Heptylcyclopentanon; 2-Pentyl-cyclopentanon; 2-Hydroxy-3-methyl-2-cyclopenten-1-on; 3-Methyl-cis-2-penten-1-yl-2-cyclopenten-1-on; 3-Methyl-2-pentyl-2-cyclopenten-1-on; 3-Methyl-4-cyclopentadecenon; 3-Methyl-5-cyclopentadecenon; 3-Methylcyclopenta-decanon; 4-(1-Ethoxyvinyl)-3,3,5,5-tetramethylcyclohexanon; 4-tert.-Pentylcyclo-hexanon; 5-Cyclohexadecen-1-on; 6,7-Dihydro-1,1,2,3,3-pentamethyl-4(5H)-indanon; 8-Cyclohexadecen-1-on; 9-Cycloheptadecen-1-on; Cyclopentadecanon; Cyclohexadecanon;
der cycloaliphatischen Aldehyde wie z. B. 2-Methyl-4-(2,2,6-trimethyl-cyclohexen-1-yl)-2-butenal; 4-(4-Hydroxy-4-methylpentyl)-3-cyclohexencarbaldehyd; 4-(4-Methyl-3-penten-1-yl)-3-cyclohexencarbaldehyd;
der cycloaliphatischen Ketone wie z. B. 1-(3,3-Dimethylcyclohexyl)-4-penten-1-on; 2,2-Dimethyl-1-(2,4-dimethyl-3-cyclohexen-1-yl)-1-propanon; 1-(5,5-Dimethyl-1-cyclohexen-1-yl)-4-penten-1-on; 2,3,8,8-Tetramethyl-1,2,3,4,5,6,7,8-octahydro-2-naphtalenylmethylketon; Methyl-2,6,10-trimethyl-2,5,9-cyclododecatrienylketon; tert.-Butyl-(2,4-dimethyl-3-cyclohexen-1-yl)keton;
der Ester cyclischer Alkohole wie z. B. 2-tert-Butylcyclohexylacetat; 4-tert-Butylcyclohexylacetat; 2-tert-Pentylcyclohexylacetat; 4-tert-Pentyl-cyclo-hexyl-acetat; 3,3,5-Trimethylcyclohexylacetat; Decahydro-2-naphthylacetat;2-Cyclopentylcyclopentylcrotonat; 3-Pentyltetrahydro-2H-pyran-4-ylacetat; Decahydro-2,5,5,8a-tetramethyl-2-naphthylacetat; 4,7-Methano-3a,4,5,6,7,7a-hexa-hydro-5, bzw. 6-indenylacetat; 4,7-Methano-3a,4,5,6,7,7a-hexahydro-5, bzw. 6-indenyl-propionat; 4,7-Methano-3a,4,5,6,7,7a-hexahydro-5, bzw. 6-inden-ylisobutyrat; 4,7-Methanooctahydro-5, bzw. 6-indenylacetat;
der Ester cycloaliphatischer Alkohole wie z. B.1-Cyclohexylethylcrotonat;
der Ester cycloaliphatischer Carbonsäuren wie z. B. Allyl-3-cyclohexylpropionat; Allylcyclohexyloxyacetat; cis- und trans-Methyldihydrojasmonat; cis- und trans-Methyljasmonat; Methyl-2-hexyl-3-oxocyclopentancarboxylat; Ethyl-2-ethyl-6,6-dimethyl-2-cyclohexencarboxylat; Ethyl-2,3,6,6-tetramethyl-2-cyclohexen-carboxylat; Ethyl-2-methyl-1,3-dioxolan-2-acetat;
der araliphatischen Alkohole wie z. B. Benzylalkohol; 1-Phenylethylalkohol; 3-Phenylpropanol; 2-Phenylpropanol; 2-Phenoxyethanol; 2,2-Dimethyl-3-phenylpropanol; 2,2-Dimethyl-3-(3-methylphenyl)propanol; 1,1-Dimethyl-2-phenylethylalkohol; 1,1-Dimethyl-3-phenylpropanol; 1-Ethyl-1-methyl-3-phenylpropanol; 2-Methyl-5-phenylpentanol; 3-Methyl-5-phenylpentanol; 3-Phenyl-2-propen-1-ol; 4-Methoxybenzylalkohol; 1-(4-Isopropylphenyl)ethanol;
der Ester von araliphatischen Alkoholen und aliphatischen Carbonsäuren wie z. B. Benzylacetat; Benzylpropionat; Benzylisobutyrat; Benzylisovalerianat; 2-Phenylethylacetat; 2-Phenylethylpropionat; 2-Phenylethylisobutyrat; 2-Phenylethylisovalerianat; 1-Phenylethylacetat; alpha-Trichlormethylbenzylacetat; alpha, alpha-Dimethylphenylethylacetat; alpha,alpha-Dimethylphenylethylbutyrat; Cinnamylacetat; 2-Phenoxyethylisobutyrat; 4-Methoxybenzylacetat;
der araliphatischen Ether wie z. B. 2-Phenylethylmethylether; 2-Phenylethylisoamylether; 2-Phenylethyl-1-ethoxyethylether; Phenylacetaldehyddimethylacetal; Phenylacetaldehyddiethylacetal; Hydratropaaldehyddimethylacetal; Phenylacetaldehydglycerinacetal; 2,4,6-Trimethyl-4-phenyl-1,3-dioxan; 4,4a,5,9b-Tetrahydroindeno[1,2-d]-m-dioxin; 4,4a,5,9b-Tetrahydro-2,4-dimethylindeno[1,2-d]-m-dioxin;
der aromatischen und araliphatischen Aldehyde wie z. B. Benzaldehyd; Phenylacetaldehyd; 3-Phenylpropanal; Hydratropaaldehyd; 4-Methylbenzaldehyd; 4-Methylphenylacetaldehyd; 3-(4-Ethylphenyl)-2,2-dimethylpropanal; 2-Methyl-3-(4-isopropylphenyl)propanal; 2-Methyl-3-(4-isobutylphenyl)propanal; 3-(4-tert.-Butylphenyl)propanal; Zimtaldehyd; alpha-Butylzimtaldehyd; alpha-Hexylzimtaldehyd; 3-Methyl-5-phenylpentanal; 4-Methoxybenzaldehyd; 4-Hydroxy-3-methoxybenzaldehyd; 4-Hydroxy-3-ethoxybenzaldehyd; 3,4-Methylendioxybenzaldehyd; 3,4-Dimethoxybenzaldehyd; 2-Methyl-3-(4-methoxyphenyl)propanal; 2-Methyl-3-(4-methylendioxyphenyl)propanal;
der aromatischen und araliphatischen Ketone wie z. B. Acetophenon; 4-Methylacetophenon; 4-Methoxyacetophenon; 4-tert.-Butyl-2,6-dimethylaceto-phenon; 4-Phenyl-2-butanon; 4-(4-Hydroxyphenyl)-2-butanon; 1-(2-Naphtha-lenyl)ethanon;2-Benzofuranylethanon;(3-Methyl-2-benzofuranyl)ethanon; Benzophenon; 1,1,2,3,3,6-Hexamethyl-5-indanylmethylketon; 6-tert.-Butyl-1,1-di-methyl-4-indanylmethylketon; 1-[2,3-dihydro-1,1,2,6-tetramethyl-3-(1-methylethyl) -1H-5-indenyl]ethanon; 5',6',7',8'-Tetrahydro-3',5',5',6',8',8'-hexamethyl-2-aceto-naphthon;
der aromatischen und araliphatischen Carbonsäuren und deren Ester wie z. B. Benzoesäure; Phenylessigsäure; Methylbenzoat; Ethylbenzoat; Hexylbenzoat; Benzylbenzoat; Methylphenylacetat; Ethylphenylacetat; Geranylphenylacetat; Phenylethylphenylacetat; Methylcinnamat; Ethylcinnamat; Benzylcinnamat; Phenylethylcinnamat; Cinnamylcinnamat; Allylphenoxyacetat; Methylsalicylat; Hexylsalicylat; Cyclohexylsalicylat; Cis-3-Hexenylsalicylat; Benzylsalicylat; Phenylethylsalicylat; Methyl-2,4-dihydroxy-3,6-dimethylbenzoat; Ethyl-3-phenylglycidat; Ethyl-3-methyl-3-phenylglycidat;
der stickstoffhaltigen aromatischen Verbindungen wie z. B. 2,4,6-Trinitro-1,3-dimethyl-5-tert.-butylbenzol; 3,5-Dinitro-2,6-dimethyl-4-tert.-butylacetophenon; Zimtsäurenitril; 3-Methyl-5-phenyl-2-pentensäurenitril; 3-Methyl-5-phenylpentan-säurenitril; Methylanthranilat; Methy-N-methylanthranilat; Schiff'sche Basen von Methylanthranilat mit 7-Hydroxy-3,7-dimethyloctanal, 2-Methyl-3-(4-tert.-butyl-phenyl)propanal oder 2,4-Dimethyl-3-cyclohexencarbaldehyd; 6-Isopropyl-chinolin; 6-Isobutylchinolin; 6-sec.-Butylchinolin;2-(3-Phenylpropyl)pyridin; Indol; Skatol; 2-Methoxy-3-isopropylpyrazin; 2-Isobutyl-3-methoxypyrazin;
der Phenole, Phenylether und Phenylester wie z. B. Estragol; Anethol; Eugenylmethylether; Isoeugenol; Isoeugenylmethylether; Thymol; Carvacrol; Diphenylether; beta-Naphthylmethylether; beta-Naphthylethylether; beta-Naphthyliso-butylether; 1,4-Dimethoxybenzol; Eugenylacetat; 2-Methoxy-4-methylphenol; 2-Ethoxy-5-(1-propenyl)phenol; p-Kresylphenylacetat;
der heterocyclischen Verbindungen wie z. B. 2,5-Dimethyl-4-hydroxy-2H-furan-3-on; 2-Ethyl-4-hydroxy-5-methyl-2H-furan-3-on; 3-Hydroxy-2-methyl-4H-pyran-4-on; 2-Ethyl-3-hydroxy-4H-pyran-4-on;
der Lactone wie z. B. 1,4-Octanolid; 3-Methyl-1,4-octanolid; 1,4-Nonanolid; 1,4-Decanolid; 8-Decen-1,4-olid; 1,4-Undecanolid; 1,4-Dodecanolid; 1,5-Decanolid; 1,5-Dodecanolid;4-Methyl-1,4-decanolid; 1,15-Pentadecanolid; 1,16-Hexadeca-nolid; 9-Hexadecen-1,16-olid; 10-Oxa-1,16-hexadecanolid; 11-Oxa-1,16-hexa-decanolid; 12-Oxa-1,16-hexadecanolid; Ethylen-1,12-dodecandioat; Ethylen-1,13-tridecandioat; 2,3-Dihydrocumarin; Octahydrocumarin.

Im Rahmen der erfindungsgemäßen Verwendungen der erfindungsgemäßen Mischungen als Riech- oder Aromastoffmischung (insbesondere mit ambrierenden Note) betrifft die vorliegende Erfindung auch die Verwendung zum Versehen von (a) Haaren, (b) Haut oder (c) textilen Fasern mit einem - insbesondere ambrierenden Duft (hinsichtlich weiterer Geruchs- bzw. Geschmacksnoten s. oben). Die vorliegende Erfindung betrifft auch entsprechende Verfahren und (vorzugsweise tensidhaltige) Mischungen. Gemäß einem verwandten Aspekt betrifft die vorliegende Erfindung auch die Verwendung der erfindungsgemäßen Mischungen umfassend Verbindungen der Formeln (I) und (II) als Mittel zum Erhöhen der Substantivität und/oder Retention einer Riechstoffmischung und/oder als Fixateur und/oder als Mittel zum Erhöhen des über einer tensidhaltigen wässrigen Lösung wahrgenommenen Geruches anderer Riechstoffe.

Die erfindungsgemäßen Mischungen sind auch hervorragende Booster (Verstärker; Enhancer) für andere Riechstoffe, d.h. sie verstärken die sensorische Wirkung dieser anderen Riechstoffe. Zu den zusätzlichen positiven Sekundäreigenschaften der erfindungsgemäßen Mischungen zählen neben der bereits genannten hohen Stabilität unter bestimmten Anwendungsbedingungen auch eine hohe Ausgiebigkeit, ein gutes Haftungsvermögen sowie eine hohe Substantivität.

Die vorliegende Erfindung betrifft auch ein parfümiertes oder aromatisiertes Produkt, umfassend
(A) einen festen oder halbfesten Träger und eine den festen bzw. halbfesten Träger kontaktierende sensorisch wirksame Menge einer erfindungsgemäßen Mischung
   oder
(B) eine flüssige Phase und darin gelöst oder suspendiert oder damit verdünnt eine sensorisch wirksame Menge einer erfindungsgemäßen Mischung.

Hinsichtlich der bevorzugten Ausgestaltung der in einem erfindungsgemäßen parfümierten oder aromatisierten Produkt einzusetzenden erfindungsgemäßen Mischung gilt das oben Gesagte.

Eine erfindungsgemäße Mischung lässt sich beispielsweise herstellen, indem eine Vormischung der Komponenten (a) und (b) bereitgestellt und diese Vormischung mit einem oder mehreren weiteren Riech- oder Aromastoffen (als Komponente (c)) vermischt wird. Die Komponenten (a) und (b) werden dabei regelmäßig in einer Gesamtmenge eingesetzt, die ausreicht, in der fertigen Mischung eine Geruchs- oder Geschmacksnote des Typs strahlend-ambrierend, exaltierend, weich holzig zu vermitteln, zu modifizieren und/oder zu verstärken.

Eine erfindungsgemäße Mischung umfasst vorzugsweise eine Gesamtmenge der Komponenten (a) und (b) im Bereich von 0,00001 bis 99,9 Gew.-%, vorzugsweise 0,001 bis 70 Gew.-% und besonders bevorzugt 0,01 bis 50 Gew.-%, bezogen auf die Gesamtmenge an Riech- oder Aromastoffen in der Mischung.

Sofern die erfindungsgemäß in Kombination miteinander einzusetzenden Komponenten (a) und (b) hauptsächlich eingesetzt werden, um einer Riech- oder Aromastoffkomposition mehr (Aus)Strahlung, Abrundung und/oder Harmonie zu verleihen und/oder bestimmte Noten zu verstärken, liegt die Gesamtmenge der Komponenten (a) und (b) vorzugsweise vergleichsweise niedrig und besonders bevorzugt im Bereich von 0,01 bis 5 Gew.-%, bevorzugt im Bereich von 0,1 bis 2 Gew.-%, bezogen auf die Gesamtmenge an Riech- oder Aromastoffen in der Mischung. Sofern innerhalb der bevorzugten Konzentrationsbereiche eine vergleichsweise niedrige Konzentration gewählt wird, kommt es in Abhängigkeit von den weiteren Komponenten der jeweiligen Mischung in manchen Fällen noch nicht zu der Vermittlung der oben angegebenen Eigengeruchs- oder -geschmacksnoten.

Die Herstellung erfindungsgemäß einzusetzender Ether der Formeln (I) bzw. (II) kann mittels an sich bekannter Verfahren erfolgen.

So können beispielsweise die alpha-Ambrinolalkylether der Formel (I) durch Alkylierung des alpha-Ambrinol mit Alkylhalogenid hergestellt werden, analog der Vorschriften in WO 82/00030. So kann insbesondere die Synthese von alpha-Ambrinolmethylether (Verbindung der Formel (I) mit R = Methyl) durch Umsetzung von alpha-Ambrinol mit Methyljodid erfolgen (siehe das folgende Formelschema).

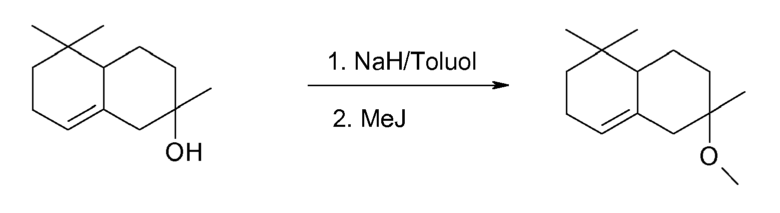

Die Ether der Formel (II) können beispielsweise ausgehend von alpha- Dihydrojonon gemäß den Vorschriften aus US 2,803,662 (Umsetzung mit Alkohol in Gegenwart von starker Säure) oder EP 0330 995 (z. B. Umsetzung mit Orthoameisensäure-Alkylester in Gegenwart von Bortrifluorid-Etherat) erhalten werden. Hinsichtlich beispielhafter Verfahrensgestaltungen sei auf die besagten Dokumente verwiesen. Ein beispielhaftes Reaktionsschema mit beispielhaften Angaben zu bevorzugten Umsetzungspartnern für das Edukt alpha-Dihydrojonon ist nachfolgend angegeben.

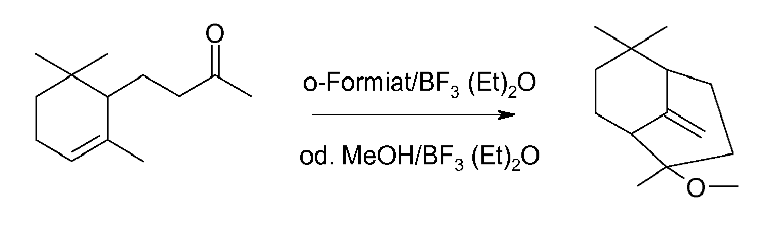

Überraschenderweise lassen sich die erfindungsgemäß einzusetzenden Verbindungen der Formel (I) 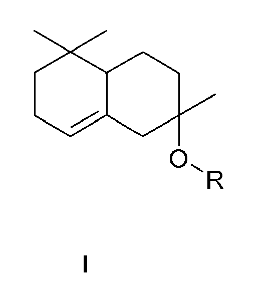 wobei
R in Formel (I) ausgewählt ist aus der Gruppe bestehend aus Methyl und Ethyl, durch ein Verfahren mit folgendem Schritt herstellen:
   Umsetzen von gamma-Dihydrojonon mit
      (a) einem Alkohol-Precursor wie z.B. Orthoformiat der Formel HC(OR)₃, wobei R die gleiche Bedeutung besitzt wie in Formel (I) oder einem Alkohol der Formel ROH, wobei R die gleiche Bedeutung besitzt wie in Formel (I)
         in Gegenwart von
      (b) einer starken Säure.

Die Verfahrensführung ähnelt somit derjenigen aus den Dokumenten US 2,803,662 und EP 0 330 995, mit dem Unterschied, dass gamma-Dihydrojonon als Edukt eingesetzt wird.

Die im erfindungsgemäßen Verfahren zur Herstellung einer Verbindung der Formel (I) eingesetzte starke Säure ist vorzugsweise eine Protonensäure oder eine Lewissäure. Vorzugsweise ist die starke Säure ausgewählt aus der Gruppe bestehend aus: Chlorwasserstoffgas, Salzsäure, Schwefelsäure, Phosphorsäure, Trifluoressigsäure, Methansulfonsäure, p-Toluolsulfonsäure, Camphersulfonsäure, BF₃, BF₃(Et₂)O, BF₃-H₃PO₄ und saure lonenaustauscher.

Die gemäß dem erfindungsgemäßen Verfahren hergestellte Verbindung der Formel (I) kann nun zur Herstellung einer erfindungsgemäßen Mischung mit einer Verbindung der Formel (II) vermischt werden. Vorzugsweise wird jedoch gleichzeitig mit der Umsetzung des gamma-Dihydrojonons und durch Umsetzung mit den gleichen Reaktionspartnern (siehe die obigen Reaktionspartner (a) und (b)) alpha-Dihydrojonon zu einer Verbindung der Formel (II) umgesetzt 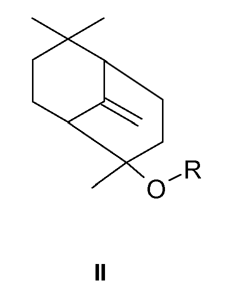 wobei R in Formel (II) die gleiche Bedeutung besitzt wie in Formel (I).

In der Praxis wird somit vorzugsweise ein Gemisch von alpha-Dihydrojonon und gamma-Dihydrojonon den Verfahrensbedingungen unterworfen, die in der US 2,803,662 und der EP 0 330 995 im Wesentlichen beschrieben sind. Unter diesen Verfahrensbedingungen bildet sich aus dem alpha-Dihydrojonon in bekannter Weise das exo/endo-Gemisch der Verbindungen der Formel (II) (wobei die Verfahrensbedingungen vorteilhafterweise so eingestellt sind, dass die exo-Form überwiegt); gleichzeitig bildet sich aus dem gamma-Dihydrojonon die Verbindung der Formel (I).

Das resultierende Produkt der gleichzeitigen Umsetzung von alpha-Dihydrojonon und gamma-Dihydrojonon ist eine erfindungsgemäße Mischung, die nachfolgend als Syntheseproduktmischung bezeichnet wird. Vorzugsweise ist eine erfindungsgemäße Mischung eine gegebenenfalls aufgereinigte Syntheseproduktmischung, die eine Gesamtmenge von zumindest 90 Gew.-%, vorzugsweise zumindest 95 Gew.-% an Verbindungen der Formeln (I) und (II) umfasst.

Dass es (i) möglich ist, ausgehend von gamma-Dihydrojonon die erfindungsgemäßen Verbindungen der Formel (I) herzustellen, dass (ii) die Umsetzung unter Verfahrensbedingungen erfolgen kann, die sich im Wesentlichen aus den Dokumenten US 2,803,662 und der EP 0 330 995 ergeben, und dass (iii) gamma-Dihydrojonon und alpha-Dihydrojonon in einem Reaktionssatz umgesetzt werden können und ohne gegenseitige Störung zu Verbindungen der Formel (I) und Verbindungen der Formel (II) führen, ist überraschend.

Diese überraschenden Möglichkeiten der Herstellung einer erfindungsgemäßen Mischung umfassend Verbindungen der Formeln (I) und (II) bedeuten, dass es besonders einfach ist, die erfindungsgemäßen Mischungen herzustellen; die erfindungsgemäße Herstellung einer Verbindung der Formel (I) und einer Verbindung der Formel (II) mit jeweils gleicher Bedeutung von R in einem Reaktionssatz ist somit erfindungsgemäß besonders bevorzugt; die separate Herstellung von Verbindungen der Formel (I) und Verbindungen der Formel (II) ist im Unterschied dazu nicht sonderlich bevorzugt, da es einen zusätzlichen Aufwand erfordern würde, die einzeln hergestellten Ether zu isolieren und anschließend zu mischen.

Abhängig vom gewählten Reaktionsansatz und der konkreten Verfahrensgestaltung ist es möglich, die Anteile der Verbindungen der Formel (I) und Verbindungen der Formel (II) in der Syntheseproduktmischung zu variieren und gezielt einzustellen. Zu bevorzugten Herstellverfahren, die unmittelbar zu Syntheseproduktmischungen führen, welche Verbindungen der Formel (I) neben Verbindungen der Formel (II) umfassen, vergleiche die Beispiele.

Das nachfolgende Formelschema illustriert beispielhaft die erfindungsgemäßen Verfahrensgestaltungen; das Formelschema bezieht sich vereinfachend lediglich auf Verbindungen der Formeln (I) und (II) mit R = Methyl; für R = Ethyl gilt aber natürlich entsprechendes:

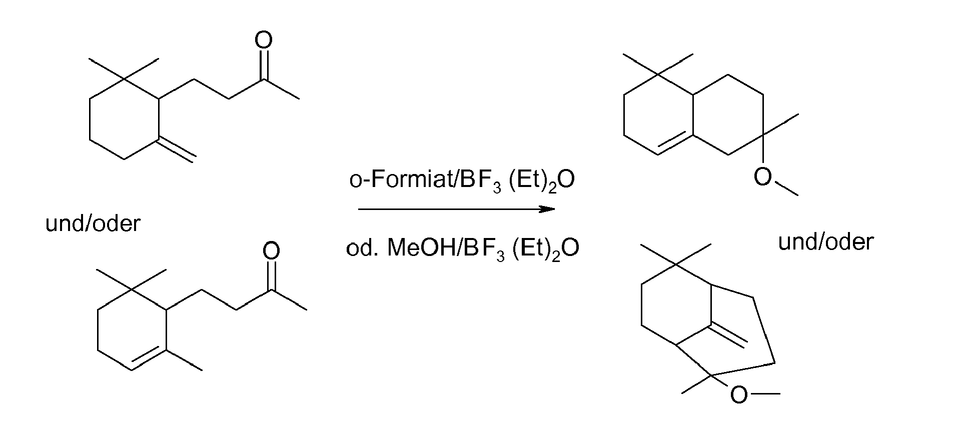

Erfindungsgemäße Mischungen, welche Verbindungen der Formeln (I) und (II) enthalten, können in flüssiger Form unverdünnt oder mit einem Lösungsmittel verdünnt zur Parfümierung oder Aromatisierung eingesetzt werden; bei Anwesenheit eines Lösungsmittels liegt ein erfindungsgemäßes Produkt vor, welches das Lösungsmittel als flüssige Phase umfasst. Geeignete Lösungsmittel sind z. B. Ethanol, Isopropanol, Diethylenglycolmonoethylether, Glycerin, Propylenglycol, 1,2-Butylenglycol, Dipropylenglycol, Diethylphtalat, Triethylcitrat, Isopropylmyristat, Triacetin usw.

Des Weiteren können erfindungsgemäße Mischungen, welche Verbindungen der Formeln (I) und (II) enthalten, an einem Trägerstoff adsorbiert sein, der sowohl für eine feine Verteilung der Riech- oder Aromastoffe im Produkt als auch für eine kontrollierte Freisetzung bei der Anwendung sorgt. Derartige Träger können poröse anorganische Materialien wie Leichtsulfat, Kieselgele, Zeolithe, Gipse, Tone, Tongranulate, Gasbeton usw. oder organische Materialien wie Hölzer, Cellulose-basierende Stoffe, Zucker, Dextrine (z. B. Maltodextrin) oder Kunststoffe wie PVC, Polyvinylacetate oder Polyurethane sein. Bei Anwesenheit eines festen oder halbfesten Trägers liegt ein erfindungsgemäßes parfümiertes oder aromatisiertes Produkt vor, so dass die den Träger kontaktierende Menge der erfindungsgemäßen Mischung sensorisch wirksam ist.

Erfindungsgemäße Mischungen können auch mikroverkapselt, sprühgetrocknet, als Einschluss-Komplexe oder als Extrusions-Produkte (d.h. als erfindungsgemäße Produkte) vorliegen und in dieser Form z. B. einem zu parfümierenden oder aromatisierenden Produkt hinzugefügt werden.

Gegebenenfalls können die Eigenschaften der derart modifizierten Mischungen durch sog. "Coaten" mit geeigneten Materialien im Hinblick auf eine gezieltere Duftfreisetzung weiter optimiert werden, wozu vorzugsweise wachsartige Kunststoffe wie z. B. Polyvinylalkohol verwendet werden. Die resultierenden Produkte stellen wiederum erfindungsgemäße parfümierte oder aromatisierte Produkte dar.

Die Mikroverkapselung der erfindungsgemäßen Mischungen zu erfindungsgemäßen Produkten kann beispielsweise durch das sogenannte Koazervationsverfahren mit Hilfe von Kapselmaterialien z. B. aus polyurethanartigen Stoffen oder Weichgelatine, erfolgen. Die sprühgetrockneten erfindungsgemäßen Mischungen können beispielsweise durch Sprühtrocknung einer die erfindungsgemäße Mischung enthaltenden Emulsion, bzw. Dispersion hergestellt werden, wobei als Trägerstoffe modifizierte Stärken, Proteine, Dextrin und pflanzliche Gummen verwendet werden können. Einschluss-Komplexe können z. B. durch Eintragen von Dispersionen von der Riech-oder Aromastoffkomposition und Cyclodextrinen oder Harnstoffderivaten in ein geeignetes Lösungsmittel, z. B. Wasser, hergestellt werden. Extrusions-Produkte können durch Verschmelzen der erfindungsgemäßen Mischungen mit einem geeigneten wachsartigen Stoff und durch Extrusion mit nachfolgender Erstarrung, ggf. in einem geeigneten Lösungsmittel, z. B. Isopropanol, erhalten werden.

Erfindungsgemäße Mischungen können in konzentrierter Form in Lösungen oder in obern beschriebener modifizierter Form verwendet werden für die Herstellung von erfindungsgemäßen parfümierten oder aromatisierten Produkten wie z. B. Parfüm-Extraits, Eau de Parfums, Eau de Toilettes, Rasierwässer, Eau de Colognes, Pre-shave-Produkte, Splash-Colognes und parfümierten Erfrischungstüchern sowie die Parfümierung von sauren, alkalischen und neutralen Reinigungsmitteln, wie z. B. Fußbodenreinigern, Fensterglasreinigern, Geschirrspülmittel, Bad- und Sanitärreinigern, Scheuermilch, festen und flüssigen WC-Reinigern, pulver- und schaumförmigen Teppichreinigern, Textilerfrischern, Bügelhilfen, flüssigen Waschmitteln, pulverförmigen Waschmitteln, Wäschevorbehandlungsmitteln wie Bleichmittel, Einweichmittel und Fleckenentfernern, Wäscheweichspülern, Waschseifen, Waschtabletten, Desinfektionsmitteln, Oberflächendesinfektionsmitteln sowie von Luftverbesserern in flüssiger, gelartiger oder auf einem festen Träger aufgebrachter Form, Aerosolsprays, Wachsen und Polituren wie Möbelpolituren, Fußbodenwachsen, Schuhcremes sowie Körperpflegemitteln wie z. B. festen und flüssigen Seifen, Duschgelen, Shampoos, Rasierseifen, Rasierschäumen, Badeölen, kosmetischen Emulsionen vom Öl-in-Wasser-, vom Wasser-in-Öl- und vom Wasser-in-Öl-in-Wasser-Typ wie z. B. Hautcremes- und -lotionen, Gesichtscremes und -lotionen, Sonnenschutzcremes-und - lotionen, After-sun-cremes und -lotionen, Handcremes und -lotionen, Fußcremes und -lotionen, Enthaarungscremes und -lotionen, After-shave-Cremes und -lotionen, Bräunungscremes und -lotionen, Haarpflegeprodukten wie z. B. Haarsprays, Haargelen, festigenden Haarlotionen, Haarspülungen, permanenten und semipermanenten Haarfärbemitteln, Haarverformungsmitteln wie Kaltwellen und Haarglättungsmitteln, Haarwässern, Haarcremes und -lotionen, Deodorantien und Antiperspirantien wie z. B. Achselsprays, Roll-ons, Deosticks, Deocremes, Produkten der dekorativen Kosmetik wie z. B. Lidschatten, Nagellacke, Make-ups, Lippenstifte, Mascara sowie von Kerzen, Lampenölen, Räucherstäbchen, Insektiziden, Repellentien und Treibstoffen.

Die erfindungsgemäßen Mischungen können in aromatisierte oder zu aromatisierende Artikel eingearbeitet werden, insbesondere in der Ernährung, der Mundpflege oder dem Genuss dienende Zubereitungen.

Der Ernährung oder dem Genuss dienende Zubereitungen sind z. B. Backwaren (z. B. Brot, Trockenkekse, Kuchen, sonstiges Gebäck), Süßwaren (z. B. Schokoladen, Schokoladenriegelprodukte, sonstige Riegelprodukte, Fruchtgummi, Hart- und Weichkaramellen, Kaugummi), alkoholische oder nicht-alkoholische Getränke (z. B. Kaffee, Tee, Wein, weinhaltige Getränke, Bier, bierhaltige Getränke, Liköre, Schnäpse, Weinbrände, fruchthaltige Limonaden, isotonische Getränke, Erfrischungsgetränke, Nektare, Obst- und Gemüsesäfte, Frucht- oder Gemüsesaftzubereitungen), Instantgetränke (z. B. Instant-Kakao-Getränke, Instant-Tee-Getränke, Instant-Kaffeegetränke), Fleischprodukte (z. B. Schinken, Frischwurst- oder Rohwurstzubereitungen, gewürzte oder marinierte Frisch- oder Pökelfleischprodukte), Eier oder Eiprodukte (Trockenei, Eiweiß, Eigelb), Getreideprodukte (z. B. Frühstückscerealien, Müsliriegel, vorgegarte Fertigreis-Produkte), Milchprodukte (z. B. Milchgetränke, Milcheis, Joghurt, Kefir, Frischkäse, Weichkäse, Hartkäse, Trockenmilchpulver, Molke, Butter, Buttermilch, teilweise oder ganz hydrolisierte Milchprotein-haltige Produkte), Produkte aus Sojaprotein oder anderen Sojabohnen-Fraktionen (z. B. Sojamilch und daraus gefertigte Produkte, Sojalecithin-haltige Zubereitungen, fermentierte Produkte wie Tofu oder Tempe oder daraus gefertigte Produkte, Sojasoßen), Fruchtzubereitungen (z. B. Konfitüren, Fruchteis, Fruchtsoßen, Fruchtfüllungen), Gemüsezubereitungen (z. B. Ketchup, Soßen, Trockengemüse, Tiefkühlgemüse, vorgegarte Gemüse, in Essig eingelegte Gemüse, eingekochte Gemüse), Knabberartikel (z. B. gebackene oder frittierte Kartoffelchips oder Kartoffelteigprodukte, Brotteigprodukte, Extrudate auf Mais- oder Erdnussbasis), Produkte auf Fett- und Ölbasis oder Emulsionen derselben (z. B. Mayonnaise, Remoulade, Dressings, Würzzubereitungen), sonstige Fertiggerichte und Suppen (z. B. Trockensuppen, Instant-Suppen, vorgegarte Suppen), Gewürze, Würzmischungen sowie insbesondere Aufstreuwürzungen (englisch: Seasonings), die beispielsweise im Snackbereich Anwendung finden. Nach Einarbeiten der erfindungsgemäßen Mischungen sind diese Zubereitungen erfindungsgemäße Zubereitungen (als Beispiel erfindungsgemäßer aromatisierter Produkte).

Erfindungsgemäße Zubereitungen können z. B. als Halbfertigware oder als Würzmischung vorliegen.

Erfindungsgemäße Zubereitungen können insbesondere als Halbfertigware zur Herstellung weiterer der Ernährung oder dem Genuss dienenden Zubereitungen dienen, insbesondere in sprühgetrockneter Form. Erfindungsgemäße Zubereitungen können auch in Form von Kapseln, Tabletten (nichtüberzogene sowie überzogene Tabletten, z. B. magensaftresistente Überzüge), Dragees, Granulaten, Pellets, Feststoffmischungen, Dispersionen in flüssigen Phasen, als Emulsionen, als Pulver, als Lösungen, als Pasten oder als andere schluck- oder kaubare Zubereitungen als Nahrungsergänzungsmittel vorliegen.

Der Mundpflege dienende erfindungsgemäße Zubereitungen sind insbesondere Mund- und/oder Zahnpflegemittel wie Zahnpasten, Zahngele, Zahnpulver, Mundwässer, Kaugummis und andere Mundpflegemittel.

Weitere übliche Wirk-, Grund-, Hilfs- und Zusatzstoffe für der Ernährung, der Mundpflege oder dem Genuss dienende erfindungsgemäße Zubereitungen können in Mengen von 5 bis 99,999999 Gew.-%, vorzugsweise 10 bis 80 Gew.-%, bezogen auf das Gesamtgewicht der Zubereitung, enthalten sein. Ferner können die Zubereitungen Wasser in einer Menge bis zu 99,999999 Gew.-%, vorzugsweise 5 bis 80 Gew.-%, bezogen auf das Gesamtgewicht der Zubereitung, aufweisen.

Erfindungsgemäße Zubereitungen (als Beispiele erfindungsgemäßer aromatisierter Produkte), enthaltend Verbindungen der Formeln (I) und (II), werden gemäß einer bevorzugten Ausgestaltung hergestellt, indem eine erfindungsgemäße Mischung als Substanz, als Lösung (z. B. in Ethanol, Wasser oder 1,2-Propylenglycol) oder in Form eines Gemisches mit einem festen oder flüssigen Trägerstoff (z. B. Maltodextrin, Stärke, Silicagel), sonstigen Aromen oder Aromastoffen und gegebenenfalls weiteren Hilfsmitteln und/oder Stabilisatoren (z. B. natürliche oder künstliche Polysaccharide und/oder Pflanzengummen wie modifizierte Stärken oder Gummi Arabicum) in eine der Ernährung, der Mundpflege oder dem Genuss dienende Basis-Zubereitung eingearbeitet werden. Vorteilhafterweise können als Lösung und/oder Suspension oder Emulsion vorliegende erfindungsgemäße Zubereitungen auch durch Sprühtrocknung in eine feste erfindungsgemäße Zubereitung (Halbfertigware) überführt werden.

Die erfindungsgemäßen sprühgetrockneten festen Zubereitungen (als Beispiel erfindungsgemäßer Artikel) sind als Halbfertigwaren besonders gut zur Herstellung von weiteren erfindungsgemäßen Zubereitungen geeignet. In den erfindungsgemäßen sprühgetrockneten festen Zubereitungen sind vorzugsweise 50 bis 95 % Gew.-% Trägerstoffe, insbesondere Maltodextrin und/oder Stärke, 5 bis 40 % Hilfsstoffe, bevorzugt natürliche oder künstliche Polysaccharide und/oder Pflanzengummen wie modifizierte Stärken oder Gummi Arabicum enthalten.

Gemäß einer weiteren bevorzugten Ausführungsform werden zur Herstellung erfindungsgemäßer Zubereitungen eine erfindungsgemäße Mischung und gegebenenfalls andere Bestandteile der erfindungsgemäßen Zubereitung zunächst in Emulsionen, in Liposomen, z. B. ausgehend von Phosphatidylcholin, in Microsphären, in Nanosphären oder auch in Kapseln, Granulaten oder Extrudaten aus einer für Lebens- und Genussmittel geeigneten Matrix, z. B. aus Stärke, Stärkederivaten (z. B. modifizierte Stärke), Cellulose oder Cellulosederivaten (z. B. Hydroxypropylcellulose), anderen Polysacchariden (z. B. Dextrin, Alginat, Curdlan, Carageenan, Chitin, Chitosan, Pullulan), natürlichen Fetten, natürlichen Wachsen (z. B. Bienenwachs, Carnaubawachs), aus Proteinen, z. B. Gelatineoder sonstigen Naturprodukten (z. B. Schellack) eingearbeitet. Dabei können je nach Matrix die Produkte durch Sprühtrocknung, Sprühgranulation, Schmelzgranulation, Koazervation, Koagulation, Extrusion, Schmelzextrusion, Emulsionsverfahren, Beschichtung (Coating) oder andere geeignete Verkapselungsverfahren und gegebenenfalls eine geeignete Kombination der vorgenannten Verfahren erhalten werden. In einem weiteren bevorzugten Herstellungsverfahren für eine erfindungsgemäße Zubereitung wird eine erfindungsgemäße Mischung zunächst mit einem oder mehreren Komplexbildnern, beispielsweise mit Cyclodextrinen oder Cyclodextrinderivaten, bevorzugt alpha- oder gamma-Cyclodextrin, komplexiert und in dieser komplexierten Form eingesetzt.

Besonders bevorzugt ist eine erfindungsgemäße Zubereitung, bei der die Matrix so gewählt ist, dass die erfindungsgemäße Mischung von Verbindungen der Formeln (I) und (II) verzögert von der Matrix freigegeben wird, so dass eine langanhaltende Wirkung erzielt wird. Besonders bevorzugt ist insoweit eine Fett-, Wachs-, Polysaccharid-oder Proteinmatrix.

Als weitere Bestandteile für erfindungsgemäße, der Ernährung oder dem Genuss dienende Zubereitungen können übliche Grund-, Hilfs- und Zusatzstoffe für Nahrungs-oder Genussmittel verwendet werden, z. B. Wasser, Gemische frischer oder prozessierter, pflanzlicher oder tierischer Grund- oder Rohstoffe (z. B. rohes, gebratenes, getrocknetes, fermentiertes, geräuchertes und/oder gekochtes Fleisch, Knochen, Knorpel, Fisch, Gemüse, Früchte, Kräuter, Nüsse, Gemüse- oder Fruchtsäfte oder - pasten oder deren Gemische), verdauliche oder nicht verdauliche Kohlenhydrate (z. B. Saccharose, Maltose, Fructose, Glucose, Dextrine, Amylose, Amylopektin, Inulin, Xylane, Cellulose, Tagatose), Zuckeralkohole (z. B. Sorbit, Erythritol), natürliche oder gehärtete Fette (z. B. Talg, Schmalz, Palmfett, Kokosfett, gehärtetes Pflanzenfett), Öle (z. B. Sonnenblumenöl, Erdnussöl, Maiskeimöl, Olivenöl, Fischöl, Sojaöl, Sesamöl), Fettsäuren oder deren Salze (z. B. Kaliumstearat), proteinogene oder nichtproteinogene Aminosäuren und verwandte Verbindungen (z. B. γ-Aminobuttersäure, Taurin), Peptide (z. B. Glutathion), native oder prozessierte Proteine (z. B. Gelatine), Enzyme (z. B. Peptidasen), Nukleinsäuren, Nucleotide, Geschmackskorrigenzien für unangenehme Geschmackseindrücke, weitere Geschmacksmodulatoren für weitere, in der Regel nicht unangenehme Geschmackseindrücke, andere geschmacksmodulierende Stoffe (z. B. Inositolphosphat, Nucleotide wie Guanosinmonophosphat, Adenosinmonophosphat oder andere Stoffe wie Natriumglutamat oder 2-Phenoxypropionsäure), Emulgatoren (z. B. Lecithine, Diacylglycerole, Gummi arabicum), Stabilisatoren (z. B. Carageenan, Alginat), Konservierungsstoffe (z. B. Benzoesäure, Sorbinsäure), Antioxidantien (z. B. Tocopherol, Ascorbinsäure), Chelatoren (z. B. Citronensäure), organische oder anorganische Säuerungsmittel (z. B. Äpfelsäure, Essigsäure, Citronensäure, Weinsäure, Phosphorsäure), Bitterstoffe (z. B. Chinin, Coffein, Limonin, Amarogentin, Humulone, Lupolone, Catechine, Tannine), mineralische Salze (z. B. Natriumchlorid, Kaliumchlorid, Magnesiumchlorid, Natriumphosphate), die enzymatische Bräunung verhindernde Stoffe (z. B. Sulfit, Ascorbinsäure), etherische Öle, Pflanzenextrakte, natürliche oder synthetische Farbstoffe oder Farbpigmente (z. B. Carotinoide, Flavonoide, Anthocyane, Chlorophyll und deren Derivate), Gewürze, trigeminal wirksame Stoffe oder Pflanzenextrakte, enthaltend solche trigeminal wirksamen Stoffe, synthetische, natürliche oder naturidentische Aromastoffe oder Riechstoffe sowie Geruchskorrigentien.

Zahnpflegemittel (als Beispiel für der Mundpflege dienende Zubereitungen), die eine erfindungsgemäße Mischung enthalten, umfassen im Allgemeinen ein abrasives System (Schleif- oder Poliermittel), wie z. B. Kieselsäuren, Calciumcarbonate, Calciumphosphate, Aluminiumoxide und/oder Hydroxylapatite, oberflächenaktive Substanzen wie z. B. Natriumlaurylsulfat, Natriumlaurylsarcosinat und/oder Cocamidopropylbetain, Feuchthaltemitteln wie z. B. Glycerin und/oder Sorbit, Verdickungsmittel, wie z. B. Carboxymethylcellulose, Polyethylenglycole, Carrageenan und/oder Lapon ite^{®}, Süßstoffe, wie z. B. Saccharin, Geschmackskorrigenzien für unangenehme Geschmackseindrücke, Geschmackskorrigenzien für weitere, in der Regel nicht unangenehme Geschmackseindrücke, geschmacksmodulierende Stoffe (z. B. Inositolphosphat, Nucleotide wie Guanosinmonophosphat, Adenosinmonophosphat oder andere Stoffe wie Natriumglutamat oder 2-Phenoxypropionsäure), Kühlwirkstoffen wie z. B. Menthol, Mentholderivate (z. B. L-Menthol, L-Menthyllactat, L-Menthylalkylcarbonate, Menthonketale, Menthancarbonsäureamide), 2,2,2-Trialkylessigsäureamiden (z. B. 2,2-Diisopropylpropionsäuremethylamid), Icilin und Icilin-Derivate, Stabilisatoren und aktive Wirkstoffe, wie z. B. Natriumfluorid, Natriummonofluorphosphat, Zinndifluorid, quartären Ammoniumfluoriden, Zinkcitrat, Zinksulfat, Zinnpyrophosphat, Zinndichlorid, Mischungen verschiedener Pyrophosphate, Triclosan, Cetylpyridiniumchlorid, Aluminiumlactat, Kaliumcitrat, Kaliumnitrat, Kaliumchlorid, Strontiumchlorid, Wasserstoffperoxid, Aromen und/oder Natriumbicarbonat oder Geruchskorrigentien.

Kaugummis (als weiteres Beispiel für der Mundpflege dienende Zubereitungen), welche eine erfindungsgemäße Mischung enthalten, umfassen im allgemeinen eine Kaugummibase, d.h. eine beim Kauen plastisch werdende Kaumasse, Zucker verschiedener Arten, Zuckeraustauschstoffe, andere süß schmeckende Stoffe, Zuckeralkoholen, Geschmackskorrigenzien für unangenehme Geschmackseindrücke, andere Geschmacksmodulatoren für weitere, in der Regel nicht unangenehme Geschmackseindrücke, geschmacksmodulierende Stoffe (z. B. Inositolphosphat, Nucleotide wie Guanosinmonophosphat, Adenosinmonophosphat oder andere Stoffe wie Natriumglutamat oder 2-Phenoxypropionsäure), Feuchthaltemittel, Verdicker, Emulgatoren, Aromen und Stabilisatoren oder Geruchskorrigentien.

Bevorzugt können die erfindungsgemäßen Zubereitungen auch neben der erfindungsgemäßen Mischung eine Aromakomposition enthalten, um den Geschmack und/oder Geruch der Zubereitung abzurunden und zu verfeinern. Ge- eignete (zusätzliche) Aromakompositionen enthalten z. B. synthetische, natürliche oder naturidentische Aroma-, Riech- und Geschmacksstoffe sowie geeignete Hilfs- und Trägerstoffe.

Nachfolgend wird die Erfindung anhand von Beispielen näher erläutert. Sofern nicht anders angegeben, beziehen sich alle Angaben auf das Gewicht.

### Beispiel 1: Herstellung einer Mischung von Methylethern der Formeln (I) und (II) (in Anlehnung an das Verfahren gemäß US2,803,662)

In einem 2 I Rührwerk wurden 300 g Bortrifluorid-Methanol-Komplex vorgelegt und 291,4 g (1,5mol) einer Mischung aus gamma-Dihydrojonon (55%) und alpha-Dihydrojonon (40%) bei 10°C zugetropft. Anschließend wurde 15 Stunden bei RT nachgerührt. Danach wurde die Reaktionslösung mit 300 ml 10%iger NaOH vorsichtig zerlegt, die wässrige Phase abgetrennt, die organische Phase mit Wasser neutral gewaschen und eingeengt. Das Rohprodukt (312 g) wurde an einer 30 cm Vigreux-Kolonne im Vakuum fraktioniert.
Ausbeute: 234g (75% d. Th.) Sdp.: 130°-140°C/8 mbar
GC-Auswertung (20m ZB-WAX, Innendurchmesser 0,18µm / 60-9-220°C Kaltaufgabesystem):

| Index | Produkte | Gew.-% |
|---|---|---|
| 1601 | endo Methoxy-Ether der Formel II | 13,4% |
| 1631 | exo Methoxy-Ether der Formel II | 28,7 % |
| 1669 | alpha-Ambrinolmethylether | 53,9% |

### Beispiel 2: Herstellung einer Mischung von Methylethern der Formeln (I) und (II) (in Anlehnung an das Verfahren gemäß EP0330995)

In einem 2 I Rührwerk wurden 291,4 g (1,5mol) einer Mischung aus gamma-Dihydrojonon (55%) und alpha-Dihydrojonon (40%) sowie 175 g (1,66mol) Orthoameisensäuretrimethylester in 450 ml Methylenchlorid vorgelegt. Anschließend wurde innerhalb einer Stunde unter Rühren und Kühlen mit einer Lösung aus 12 g Bortrifluorid-Etherat in 150 ml Methylenchlorid versetzt. Danach wurde 1,5 Stunden unter Rückfluss nachgerührt. Nach Abkühlen auf Raumtemperatur wurde die Reaktionslösung mit 300 ml 10%iger NaOH vorsichtig zerlegt, die wässrige Phase abgetrennt, die organische Phase mit Wasser neutral gewaschen und eingeengt. Das Rohprodukt (315 g) wurde an einer 30 cm Vigreux-Kolonne im Vakuum fraktioniert.
Ausbeute: 443,5 g (92% d. Th.) Sdp.: 130°-140°C/8 mbar
GC-Auswertung (20m ZB-WAX, Innendurchmesser 0,18µm / 60-9-220°C Kaltaufgabesystem)

| Index | Produkte | Gew.-% |
|---|---|---|
| 1601 | endo Methoxy-Ether der Formel II | 9,6% |
| 1631 | exo Methoxy-Ether der Formel II | 32,3% |
| 1669 | alpha-Ambrinolmethylether | 55,9% |

### GC/MS-Daten der Methylether

### 1. endo 2-Methoxy-9-methylen-2,6,6-Trimethylbicyclo(3.3.1)nonan

MS: m/z (%) = 176 (40, M⁺ -32), 161 (37), 133 (19), 120 (16), 105 (16), 85 (100), 55 (18), 43 (9).

### 2. exo 2-Methoxy-9-methylen-2,6,6-Trimethylbicyclo(3.3.1)nonan

MS: m/z (%) = 176 (44, M⁺ -32), 161 (38), 133 (20), 120 (18), 105 (17), 91(10), 85 (100), 55 (18).

### 3. alpha- Ambrinolmethylether

MS: m/z (%) = 208 (5, M⁺), 176 (100), 161 (84), 133 (24), 120 (23), 106 (31), 105 (39), 91 (22), 85 (84).

### GC/MS-Daten der Ethylether

### 1. exo 2-Ethoxy-9-methylen-2,6,6-Trimethylbicyclo(3.3.1)nonan

MS: m/z (%) = 222 (0,2 M⁺), 176 (44), 161 (36), 133 (17), 105 (15), 99 (100), 91 (16), 71 (58), 43 (15).

### 2. endo 2-Ethoxy-9-methylen-2,6,6-Trimethylbicyclo(3.3.1)nonan

MS: m/z (%) = 222 (0,1 M⁺), 176 (38), 161 (31), 133 (15), 105 (13), 99 (100), 91 (14), 71 (57), 43 (16).

### 3. alpha- Ambrinolethylether

MS: m/z (%) = 222 (0,4 M⁺), 176 (62), 161 (42), 133 (17), 120 (16), 105 (19), 99 (100), 91 (15), 71 (47.

### Beispiel 3: Parfümkomposition (Riechstoffkomposition)

| | |
|---|---|
| Agrumex LC | 10.00 |
| Amarocit® 10%ig in DPG | 10.00 |
| Ambroxid krist. | 10.00 |
| Basilikumöl | 10.00 |
| Calone 1951 10%ig in DPG | 10.00 |
| Cedernholzöl | 10.00 |
| Cedrol Krist | 50.00 |
| Citral 10%ig in DPG | 10.00 |
| Citronellol | 5.00 |
| Cumarin | 10.00 |
| Cyclogalbanat® 10%ig in DPG | 15.00 |
| Dihydromyrcenol | 80.00 |
| Farenal® 10%ig in DPG | 5.00 |
| Galbex 10%ig in DPG | 25.00 |
| Globalide® | 80.00 |
| Globanone® | 40.00 |
| Hedion | 90.00 |
| Helional | 20.00 |
| Heliotropin | 5.00 |
| Hexenol cis-3 10%ig in DPG | 15.00 |
| Hexenylsalicylat cis-3 | 10.00 |
| Beta-Ionon | 5.00 |
| Iso E Super | 180.00 |
| Isodamascon® 10%ig in DPG | 10.00 |
| Isomuscone (Cyclohexadecanon) | 20.00 |
| Isoraldein 70 | 20.00 |
| Ketamber 10%ig in TEC | 25.00 |
| Lavandinoel Grosso Nat. | 15.00 |
| Lilial | 20.00 |
| Linalool | 20.00 |
| Linalylacetat | 40.00 |
| Mandarinenoel brasil. grün | 50.00 |
| Timberol® | 40.00 |
| Vanillin | 5.00 |
| Veloutone 10%ig in DPG | 20.00 |
| Ysamber K® | 10.00 |
| | |
| Gesamt | 1000.00 |

### DPG: Dipropylenglycol, TEC = Triethylcitrat

Geruchsbeschreibung der Parfümkomposition ohne Zusatz einer erfindungsgemäßen Mischung: frisch, holzig.

Nach Meinung der Parfümeure wird diese Parfümkomposition durch den Zusatz von 3 Gew.-% einer erfindungsgemäßen Mischung aus Beispiel 2 frischer, strahlender, abgerundeter und harmonischer, wobei eine ambrierende und süße Note hinzukommt und die holzigen und blumigen Aspekte verstärkt werden. Die eingesetzte erfindungsgemäße Mischung aus Beispiel 2 verleiht der Komposition durch ihren Eigengeruch sowie durch ihre modifizierende und verstärkende Wirkung (Booster-Wirkung) einen eigenen Charakter und verbindet die unterschiedlichen geruchlichen Elemente.

### Beispiel 4: Parfümkomposition (Riechstoffkomposition)

| | |
|---|---|
| Allylcyclohexylpropionat | 3.00 |
| Amylsalicylat | 2.00 |
| Benzylacetat | 64.00 |
| Citronellol | 122.00 |
| Citral 10% ig in DPG | 2.00 |
| Cyclamenaldehyd | 10.00 |
| Dihydromyrcenol | 3.00 |
| Dimethylbenzylcarbinylacetat | 3.00 |
| Ethylsalicylat 10% ig in DPG | 2.00 |
| Eugenol | 3.00 |
| Indoflor 10%ig in DPG | 16.00 |
| Galaxolide 50%ig in DPG | 164.00 |
| Geraniol | 35.00 |
| Dihydromethyljasmonate | 6.00 |
| Heliotropin | 4.00 |
| Hexylzimtaldehyd | 121.00 |
| Vertocitral | 4.00 |
| Hydroxycitronellal | 42.00 |
| Indol | 2.00 |
| Isobutylsalicylat | 6.00 |
| Lavandinoel Grosso Nat. | 6.00 |
| Acetylced ren | 10.00 |
| Lilial | 190.00 |
| Linalool | 35.00 |
| Linalylacetat | 10.00 |
| Methylanthranilat 10%ig in DPG | 5.00 |
| Nerol | 10.00 |
| Orangenoel | 6.00 |
| Phantolide | 4.00 |
| Phenylacetaldehyddimethylacetal | 6.00 |
| Phenylethylalkohol | 75.00 |
| Rosatol 10%ig in DPG | 6.00 |
| Sandelholzoel | 3.00 |
| Sandranol | 16.00 |
| Trifernal | 2.00 |
| Tonalid | 2.00 |
| | |
| Gesamt | 1000.00 |

### DPG: Dipropylenglycol

Geruchsbeschreibung der Parfümkomposition ohne Zusatz einer erfindungsgemäßen Mischung: blumig, Maiglöckchen.

Nach Meinung der Parfümeure erwacht diese Parfümkomposition durch den Zusatz von 6 Gew.-% einer erfindungsgemäßen Mischung gemäß Beispiel 1 zu neuem Leben. Der Eindruck von Blumigkeit wird erheblich verstärkt. Die Komposition wirkt strahlender, abgerundeter und harmonischer, wobei eine ambrierende und natürliche Note hinzukommt. Die eingesetzte erfindungsgemäße Mischung verleiht der Komposition durch ihren Eigengeruch sowie durch ihre modifizierende und verstärkende Wirkung (Booster-Wirkung) einen eigenen Charakter und verbindet die unterschiedlichen geruchlichen Elemente.

### Beispiel 5: Shampoo

Eine erfindungsgemäße Mischung aus Beispiel 1 wurde in einer Dosierung von 0,5 Gew.-% in eine Shampoo-Grundmasse folgender Zusammensetzung eingearbeitet:

| | |
|---|---|
| Natriumlaurylethersulfat (z. B. Texapon NSO, Fa. Cognis Deutschland GmbH) | 12% |
| Cocamidopropylbetain (z. B. Dehyton K, Fa. Cognis Deutschland GmbH) | 2% |
| Natriumchlorid | 1,4% |
| Citronensäure | 1,3% |
| Phenoxyethanol, Methyl-, Ethyl-, Butyl-, und Propylparaben | 0,5% |
| Wasser | 82,8% |

Der pH-Wert der Shampoo-Grundmasse lag bei etwa 6. Hieraus wurden 100 mL einer 20 Gew.%-igen wässrigen Shampoo-Lösung hergestellt. In dieser Shampoo-Lösung wurden 2 Haarsträhnchen gemeinsam für 2 Minuten gewaschen und anschließend 20 Sekunden unter fließendem handwarmen Wasser gespült. Eine Haarsträhne wurde nass in Aluminiumfolie eingepackt und die zweite Haarsträhne mit einem Fön getrocknet. Beide Haarsträhnen wurden von einem Panel geruchlich beurteilt.

Geruchsbeschreibung jeweils: sehr stark strahlend, ambrierend, exaltierend, weich holzig, erinnert an graue Ambra und Civet.

### Beispiel 6: Weichspüler

Die Parfümkomposition aus Beispiel 3 (nach Zusatz von 3 Gew.-% einer erfindungsgemäßen Mischung gemäß Beispiel 2) wurde in einer Dosierung von 0,5 Gew.-% in eine Weichspüler-Grundmasse folgender Zusammensetzung eingearbeitet:

| | |
|---|---|
| Quarternäres Ammoniummethosulfat (Esterquat), ca. 90% (z. B. Rewoquat WE 18, Fa. Witco Surfactants GmbH) | 5,5% |
| Alkyldimethylbenzylammoniumchlorid, ca. 50% (z. B. Preventol R50, Fa. Bayer AG) | 0,2% |
| Farblösung, ca. 1%-ig | 0,3% |
| | |
| Wasser | 94,0% |

Der pH-Wert der Weichspüler-Grundmasse lag im Bereich von 2 bis 3. Zwei Stofflappen wurden mit 370 g einer 1%-igen wässrigen Weichspüler-Lösung auf Basis der 0,5% Gew.-% der erfindungsgemäßen Mischung umfassenden Weichspüler-Grundmasse in einer Linetest-Maschine im Weichspülprogramm 30 Minuten bei 20°C gespült. Die Lappen wurden ausgewrungen und anschließend 20 Sekunden geschleudert. Ein Lappen wurde nass eingeschweißt, und einer zum Trocknen aufgehängt. Anschließend wurden beide Lappen durch ein Panel geruchlich beurteilt.

Geruchsbeschreibung jeweils: blumig, holzig, frisch, strahlend, ambrierende und holzige Aspekte mit leichten süßen Untertönen, abgerundeter und harmonischer Geruchseindruck.

### Beispiel 7: Waschpulver

Die Parfümölkomposition aus Beispiel 4 (nach Zusatz von 6 Gew.-% einer erfindungsgemäßen Mischung gemäß Beispiel 1) wurde in einer Dosierung von 0,4 Gew.-% in eine Waschpulver-Grundmasse der folgenden Rezeptur eingearbeitet:

| | |
|---|---|
| Lineares Na-Alkylbenzolsulfonat | 8,8 % |
| Ethoxylierter Fettalkohol C12-18 (7 EO) | 4,7 % |
| Na-Seife | 3,2 % |
| Entschäumer DOW CORNING(R) 2-4248S POWDERED ANTIFOAM, Silikonöl auf Zeolith als Trägermaterial | 3,9 % |
| Zeolith 4A | 28,3 % |
| Na-Carbonat | 11,6 % |
| Na-Salz eines Copolymers aus Acryl- und Maleinsäure (Sokalan CP5) | 2,4 % |
| Na-Silikat | 3,0 % |
| Carboxymethylcellulose | 1,2 % |
| Dequest 2066 ([[(Phosphonomethyl)imino]bis[(ethylennitrilo)bis (methylen)]]tetrakis-phosphonsäure, Natriumsalz) | 2,8 % |
| Optischer Aufheller | 0,2 % |
| Na-Sulfat | 6,5 % |
| Protease | 0,4 % |
| Natriumperborattetrahydrat | 22,0 % |
| TAED | 1,0% |

Zwei Stofflappen wurden mit 370 g einer 1%-igen wässrigen Waschpulverlauge auf Basis der 0,4 Gew.-% der Parfümölkomposition aus Beispiel 4 umfassenden Waschpulver-Grundmasse (der pH-Wert der Waschpulverlauge liegt deutlich im basischen Bereich) in einer Linetest-Maschine im Hauptwaschgang 45 Minuten bei 60°C gewaschen. Die Lappen wurden zunächst 5 Minuten mit kaltem Wasser gespült, ausgewrungen und anschließend 20 Sekunden geschleudert. Ein Lappen wurde nass eingeschweißt und einer zum Trocknen aufgehängt. Anschließend wurden beide Lappen durch ein Panel geruchlich beurteilt.

Geruchsbeschreibung jeweils: stark blumig, strahlend, ambrierende und natürliche Note mit leichten süßen und holzigen Untertönen, abgerundeter und harmonischer Geruchseindruck.

## Patentansprüche

1. Mischung umfassend oder bestehend aus
(a) einer oder mehreren Verbindungen der Formel (I) 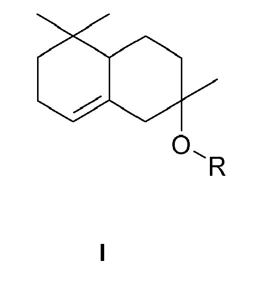 und
(b) einer oder mehreren Verbindungen der Formel (II) 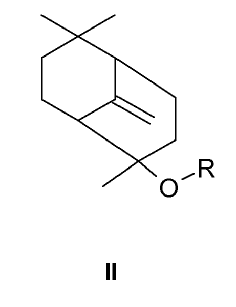 wobei
R in Formel (I) und Formel (II) die gleiche oder eine unterschiedliche Bedeutung besitzt und jeweils ausgewählt ist aus der Gruppe bestehend aus Methyl und Ethyl,
sowie gegebenenfalls
(c) einem, zwei oder mehr weiteren Riech- oder Aromastoffen.

2. Mischung nach Anspruch 1, umfassend oder bestehend aus
- jeweils einer Verbindung der Formel (I) und einer Verbindung der Formel (II) mit gleicher Bedeutung von R
sowie entweder
- einer weiteren Verbindung der Formel (I) mit der anderen Bedeutung von R
und/oder
einer weiteren Verbindung der Formel (II) mit der anderen Bedeutung von R
oder
- keiner weiteren Verbindung der Formel (I) oder (II).

3. Mischung nach Anspruch 2, umfassend oder bestehend aus
- einer Verbindung der Formel (I), in der R Methyl bedeutet, und einer Verbindung der Formel (II), in der R Methyl bedeutet.

4. Mischung nach einem der vorangehenden Ansprüche, wobei die Gesamtmenge an Verbindungen der Formel (II) in der Mischung so ausgewählt ist, dass eine ansonsten von den Verbindungen der Formel (I) verursachte animalische Geruchsnote wirksam maskiert ist.

5. Mischung nach einem der vorangehenden Ansprüche, wobei das Verhältnis der Gesamtmenge von Verbindungen der Formel (I) zur Gesamtmenge von Verbindungen der Formel (II) im Bereich von 100 : 1 bis 1 : 10 liegt, bevorzugt im Bereich von 3 : 1 bis 1 : 3, besonders bevorzugt im Bereich von 2 : 1 bis 1 : 1.

6. Mischung nach einem der vorangehenden Ansprüche, wobei die Mischung eine gegebenenfalls aufgereinigte Syntheseproduktmischung ist, die eine Gesamtmenge von zumindest 90 Gew.-%, vorzugsweise zumindest 95 Gew.-% an Verbindungen der Formeln (I) und (II) umfasst.

7. Mischung nach einem der vorangehenden Ansprüche, mit einzelnen, mehreren oder sämtlichen der folgenden geruchlichen Eigenschaften: sehr stark strahlend, ambrierend, exaltierend, weich holzig, erinnert an graue Ambra und Civet, sehr natürlicher Geruchseindruck.

8. Mischung nach einem der vorangehenden Ansprüche, wobei als Komponente (c) ein, zwei oder mehr weitere Riech- oder Aromastoffe vorhanden sind, wobei einer, zwei oder mehr dieser weiteren Riech- oder Aromastoffe eine geruchliche Eigenschaft besitzt ausgewählt aus der Gruppe bestehend aus: holzig und blumig.

9. Verwendung einer Mischung nach einem der Ansprüche 1 bis 8 als Riechstoff-oder Aromastoffmischung.

10. Verwendung nach Anspruch 9, wobei die Mischung eine Mischung nach einem der Ansprüche 4 bis 8 ist.

11. Verwendung einer Verbindung der Formel (II) 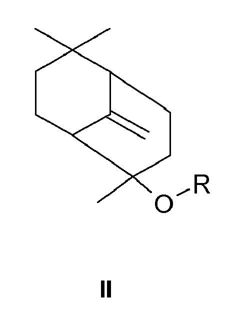 wobei
R in Formel (II) ausgewählt ist aus der Gruppe bestehend aus Methyl und Ethyl,
als Mittel zum Maskieren einer unangenehmen Geruchsnote.

12. Verwendung nach Anspruch 11 zum Maskieren der animalischen Geruchsnote einer Verbindung der Formel (I) 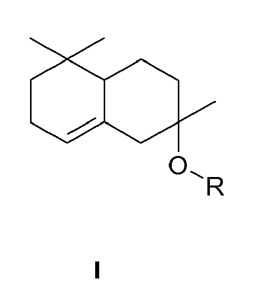 wobei
R in Formel (I) ausgewählt ist aus der Gruppe bestehend aus Methyl und Ethyl, und wobei
R in Formel (I) und Formel (II) die gleiche oder eine unterschiedliche Bedeutung besitzt.

13. Verwendung einer Verbindung der Formel (I) 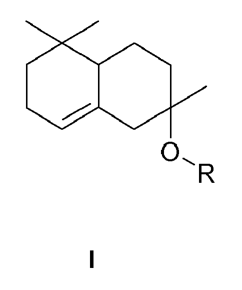 wobei
R in Formel (I) ausgewählt ist aus der Gruppe bestehend aus Methyl und Ethyl, zum Modifizieren der geruchlichen Eigenschaften einer Verbindung der Formel (II) 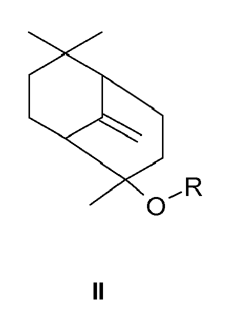
wobei
R in Formel (II) ausgewählt ist aus der Gruppe bestehend aus Methyl und Ethyl und wobei
R in Formel (I) und Formel (II) die gleiche oder eine unterschiedliche Bedeutung besitzt,
in Richtung strahlender, ambrierender.

14. Parfümiertes oder aromatisiertes Produkt, umfassend
(A) einen festen oder halbfesten Träger und
eine den festen bzw. halbfesten Träger kontaktierende sensorisch wirksame Menge einer Mischung nach einem der Ansprüche 1 bis 8,
oder
(B) eine flüssige Phase
und
darin gelöst oder suspendiert oder damit verdünnt eine sensorisch wirksame Menge einer Mischung nach einem der Ansprüche 1 bis 8.

15. Parfümiertes Produkt nach Anspruch 14, ausgewählt aus der Gruppe bestehend aus: Parfüm-Extraits, Eau de Parfums, Eau de Toilettes, Rasierwässer, Eau de Colognes, Pre-shave-Produkte, Splash-Colognes, parfümierte Erfrischungstüchern, saure, alkalische und neutrale Reinigungsmitteln, Textilerfrischern, Bügelhilfen, flüssigen Waschmitteln, pulverförmigen Waschmitteln, Wäschevorbehandlungsmitteln, Wäscheweichspülern, Waschseifen, Waschtabletten, Desinfektionsmitteln, Oberflächendesinfektionsmitteln, Luftverbesserern, Aerosolsprays, Wachse und Polituren, Körperpflegemitteln, Handcremes und -lotionen, Fußcremes und -lotionen, Enthaarungscremes und -lotionen, After-shave-Cremes und -lotionen, Bräunungscremes und -lotionen, Haarpflegeprodukten, Deodorantien und Antiperspirantien, Produkten der dekorativen Kosmetik, Kerzen, Lampenölen, Räucherstäbchen, Insektiziden, Repellentien und Treibstoffen.

16. Verfahren zum Vermitteln, Modifizieren und/oder Verstärken eines Geruches, mit folgendem Schritt:
Kontaktieren oder Vermischen eines Erzeugnisses mit einer sensorisch wirksamen Menge einer Mischung nach einem der Ansprüche 1 bis 8.

17. Verfahren zur Herstellung einer Verbindung der Formel (I) 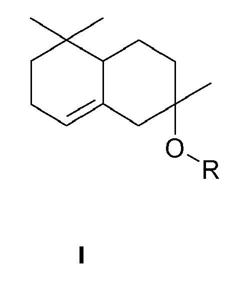 wobei
R in Formel (I) ausgewählt ist aus der Gruppe bestehend aus Methyl und Ethyl, mit folgendem Schritt:
Umsetzen von gamma-Dihydrojonon mit
(a) einem Alkohol-Precursor wie z.B. Orthoformiat der Formel HC(OR)₃, wobei R die gleiche Bedeutung besitzt wie in Formel (I)
oder
einem Alkohol der Formel ROH, wobei R die gleiche Bedeutung besitzt wie in Formel (I)
in Gegenwart von
(b) einer starken Säure.

18. Verfahren nach Anspruch 17, wobei die starke Säure eine Protonensäure oder Lewissäure ist.

19. Verfahren nach Anspruch 17 oder 18, wobei die starke Säure ausgewählt ist aus der Gruppe bestehend aus: Chlorwasserstoffgas, Salzsäure, Schwefelsäure, Phosphorsäure, Trifluoressigsäure, Methansulfonsäure, p-Toluolsulfonsäure, Camphersulfonsäure, BF₃, BF₃(Et₂)O, BF₃-H₃P0₄ und saure lonenaustauscher.

20. Verfahren nach einem der Ansprüche 17 bis 19, wobei gleichzeitig mit der Umsetzung des gamma-Dihydrojonons und durch Umsetzung mit den gleichen Reaktionspartnern alpha-Dihydrojonon zu einer Verbindung der Formel (II) umgesetzt wird 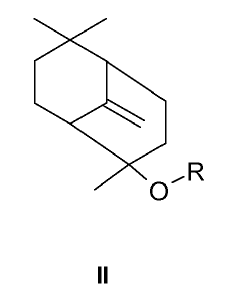 wobei R die gleiche Bedeutung besitzt wie in Formel (I).
